# EUROPEAN PATENT SPECIFICATION

(11) **EP 1 343 781 B1**
(45) Date of publication and mention of the grant of the patent: **03.09.2008**
(21) Application number: 01989898.0
(22) Date of filing: 05.12.2001
(51) Int. Cl.: C07D 403/04, C07D 401/04, C07D 401/14, A61K 31/506, A61P 29/00

(54) **INHIBITORS OF C-JUN N-TERMINAL KINASES (JNK) AND OTHER PROTEIN KINASES**
INHIBITOREN VON C-JUN N-TERMINALEN KINASEN (JNK) UND ANDEREN PROTEINKINASEN
INHIBITEURS DE KINASES C-JUN N-TERMINALES (JNK) ET D'AUTRES PROTEINES KINASES

(30) Priority: 05.12.2000 US 251409 P
(43) Date of publication of application: 17.09.2003
(73) Proprietor: VERTEX PHARMACEUTICALS INCORPORATED, Cambridge, MA 02139-4242 (US)
(72) Inventor: LEDEBOER, Mark, Acton, MA 01720 (US); SALITURO, Francesco, Marlboro, MA 01752 (US); MOON, Young-Choon, Lexington, MA 02173 (US)
(74) Representative: Cornish, Kristina Victoria Joy
(86) International application number: PCT/US2001/046383
(87) International publication number: WO 2002/046184

(56) References cited:
- WO-A-00/31063

## Description

The present invention relates to inhibitors of protein kinase, especially c-Jun N-terminal kinases (JNK), which are members of the mitogen-activated protein (MAP) kinase family. There are a number of different genes and isoforms which encode JNKs. Members of the JNK family regulate signal transduction in response to environmental stress and proinflammatory cytokines and have been implicated to have a role in mediating a number of different disorders. The invention also relates to methods for producing these inhibitors. The invention also provides pharmaceutical compositions comprising the inhibitors of the invention and methods of utilizing those compositions in the treatment and prevention of various disorders.

### BACKGROUND OF THE INVENTION

Mammalian cells respond to extracellular stimuli by activating signaling cascades that are mediated by members of the mitogen-activated protein (MAP) kinase family, which include the extracellular signal regulated kinases (ERKs), the p38 MAP kinases and the c-Jun N-terminal kinases (JNKs). MAP kinases (MAPKs) are activated by a variety of signals including growth factors, cytokines, UV radiation, and stress-inducing agents. MAPKs are serine/threonine kinases and their activation occur by dual phosphorylation of threonine and tyrosine at the Thr-X-Tyr segment in the activation loop. MAPKs phosphorylate various substrates including transcription factors, which in turn regulate the expression of specific sets of genes and thus mediate a specific response to the stimulus.

One particularly interesting kinase family are the c-Jun NH₂-terminal protein kinases, also known as JNKs. Three distinct genes, Jnk1, Jnk2, Jnk3 have been identified and at least ten different splicing isoforms of JNKs exist in mammalian cells [Gupta et al., EMBO J., 15:2760-70 (1996)]. Members of the JNK family are activated by proinflammatory cytokines, such as tumor necrosis factor-α (TNFα) and interleukin-1 β (IL-1β), as well as by environmental stress, including anisomycin, UV irradiation, hypoxia, and osmotic shock [Minden et al., Biochemica et Biophysica Acta, 1333:F85-F104 (1997)].

The down-stream substrates of JNKs include transcription factors c-Jun, ATF-2, Elk1, p53 and a cell death domain protein (DENN) [Zhang et al. Proc. Natl. Acad. Sci. USA, 95:2586-91 (1998)]. Each JNK isoform binds to these substrates with different affinities, suggesting a regulation of signaling pathways by substrate specificity of different JNKs *in vivo* (Gupta et al., *supra*).

JNKs, along with other MAPKs, have been implicated in having a role in mediating cellular response to cancer, thrombin-induced platelet aggregation, immunodeficiency disorders, autoimmune diseases, cell death, allergies, osteoporosis and heart disease. The therapeutic targets related to activation of the JNK pathway include chronic myelogenous leukemia (CML), rheumatoid arthritis, asthma, osteoarthritis, ischemia, cancer and neurodegenerative diseases.

Several reports have detailed the importance of JNK activation associated with liver disease or episodes of hepatic ischemia [Nat. Genet. 21:326-9 (1999); FEBS Lett. 420:201-4 (1997); J. Clin. Invest. 102:1942-50 (1998); Hepatology 28:1022-30 (1998)]. Therefore, inhibitors of JNK may be useful to treat various hepatic disorders.

A role for JNK in cardiovascular disease such as myocardial infarction or congestive heart failure has also been reported as it has been shown JNK mediates hypertrophic responses to various forms of cardiac stress [Circ. Res. 83:167-78 (1998); Circulation 97:1731-7 (1998); J. Biol. Chem. 272:28050-6 (1997); Circ. Res. 79:162-73 (1996); Circ. Res. 78:947-53 (1996); J. Clin. Invest. 97:508-14 (1996)].

It has been demonstrated that the JNK cascade also plays a role in T-cell activation, including activation of the IL-2 promoter. Thus, inhibitors of JNK may have therapeutic value in altering pathologic immune responses [J. Immunol. 162:3176-87 (1999); Eur. J. Immunol. 28:3867-77 (1998); J. Exp. Med. 186:941-53 (1997); Eur. J. Immunol. 26:989-94 (1996)].

A role for JNK activation in various cancers has also been established, suggesting the potential use of JNK inhibitors in cancer. For example, constitutively activated JNK is associated with HTLV-1 mediated tumorigenesis [Oncogene 13:135-42 (1996)]. JNK may play a role in Kaposi's sarcoma (KS) because it is thought that the proliferative effects of bFGF and OSM on KS cells are mediated by their activation of the JNK signaling pathway [J. Clin. Invest. 99:1798-804 (1997)]. Other proliferative effects of other cytokines implicated in KS proliferation, such as vascular endothelial growth factor (VEGF), IL-6 and TNFα, may also be mediated by JNK. In addition, regulation of the c-jun gene in p210 BCR-ABL transformed cells corresponds with activity of JNK, suggesting a role for JNK inhibitors in the treatment for chronic myelogenous leukemia (CML) [Blood 92:2450-60 (1998)].

JNK1 and JNK2 are widely expressed in a variety of tissues. In contrast, JNK3 is selectively expressed in the brain and to a lesser extent in the heart and testis [Gupta et al., *supra*; Mohit et al., Neuron 14:67-78 (1995); Martin et al., Brain Res. Mol. Brain Res. 35:47-57 (1996)]. JNK3 has been linked to neuronal apoptosis induced by kainic acid, indicating a role of JNK in the pathogenesis of glutamate neurotoxicity. In the adult human brain, JNK3 expression is localized to a subpopulation of pyramidal neurons in the CA1, CA4 and subiculum regions of the hippocampus and layers 3 and 5 of the neocortex [Mohit et al., *supra*]. The CA1 neurons of patients with acute hypoxia showed strong nuclear JNK3-immunoreactivity compared to minimal, diffuse cytoplasmic staining of the hippocampal neurons from brain tissues of normal patients [Zhang et al., *supra*]. Thus, JNK3 appears to be involved involved in hypoxic and ischemic damage of CA1 neurons in the hippocampus.

In addition, JNK3 co-localizes immunochemically with neurons vulnerable in Alzheimer's disease [Mohit et al., *supra*]. Disruption of the JNK3 gene caused resistance of mice to the excitotoxic glutamate receptor agonist kainic acid, including the effects on seizure activity, AP-1 transcriptional activity and apoptosis of hippocampal neurons, indicating that the JNK3 signaling pathway is a critical component in the pathogenesis of glutamate neurotoxicity [Yang et al., Nature, 389:865-870 (1997)].

Based on these findings, JNK signalling, especially that of JNK3, has been implicated in the areas of apoptosis-driven neurodegenerative diseases such as Alzheimer's Disease, Parkinson's Disease, ALS (Amyotrophic Lateral Sclerosis), epilepsy and seizures, Huntington's Disease, traumatic brain injuries, as well as ischemic and hemorrhaging stroke.

Recent evidence now suggests that the closely related JNK1 and JNK2 isoforms also play a role in effecting downstream apoptosis. [Tournier et al., Science, 288:870-874 (2000); Mielke et al., Neuroscience, 91:471-483 (1999)]. Therefore, these isoforms are also potentially important targets for treating stroke and other apoptosis-related diseases. For example, all three JNK isoforms (JNK1, 2 and 3) are expressed in the adult brains of humans and rodents. JNK3 is localized in the hippocampus, as mentioned above, whereas JNK1 and JNK2 are expressed in other compartments of the brain. [Derijard et al., Cell, 76:1025-1037 (1994); Gupta et al., supra; Kyriakis et al., Nature, 369:156-160 (1994)] In cells expressing JNK1 and JNK2, disruption of the Jnk1 gene caused a greater decrease in c-Jun kinase activity than disruption of the Jnk2 gene. However, both of these genes had to be knocked out in order to completely protect against stress-induced apoptosis. [Tournier et al., *supra*]. In view of these findings, it would be particularly desirable to find inhibitors that are active against both JNK1 and JNK2 targets.

There remains a high unmet medical need to develop JNK specific inhibitors that are useful in treating the various conditions associated with JNK activation, especially considering the currently available, relatively inadequate treatment options for the majority of these conditions.

Much work has been done to identify and develop drugs that inhibit MAPKs, such as p38 inhibitors. See, e.g., WO 00/31063, WO 98/52937, WO 98/27098 and WO 95/31451. However, to our knowledge, no MAPK inhibitors have been shown to be specifically selective for one or more JNKs versus other related MAPKs. Furthermore, we know of no inhibitors that are active against both JNK1 and JNK2 or against all three of the aforementioned JNK isoforms.

Accordingly, there is still a great need to develop potent inhibitors of JNKs, including JNK1, 2 and 3 inhibitors, that are useful in treating various conditions associated with JNK activation.

### SUMMARY OF THE INVENTION

It has now been found that compounds of this invention and pharmaceutical compositions thereof are effective as inhibitors of c-Jun N-terminal kinases (JNK) . These compounds are selective JNK inhibitors showing good activity against the three isoforms of JNK (JNK1, JNK2 and JNK3) and relatively low activity against p38 kinase. The compounds are therefore useful for treating JNK-mediated diseases, especially neurodegenerative diseases in which all three JNK isoforms are implicated.

### DETAILED DESCRIPTION OF THE INVENTION

The present compounds have the general formula II: 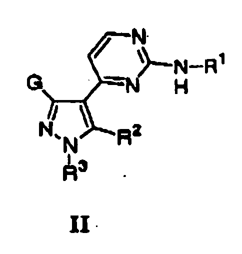 wherein:
- R¹: is selected from optionally substituted phenyl, cyclohexyl, pyridyl, naphthyl, or quinolinyl;
- R: is a C₁ to C₁₂ aliphatic or substituted aliphatic group;
- R²: is selected from hydrogen, or -R;
- R³: is selected from R, hydroxyalkyl, alkoxyalkyl, alkylthioalkyl, aminoalkyl, alkylaminoalkyl, dialkylaminoalkyl, heterocyclyl, heterocyclylalkyl, aryl, aralkyl, or aryloxyalkyl;
- G: is hydrogen or C₁₋₃ alkyl;
wherein each substitutable carbon atom of said optionally substituted phenyl, cyclohexyl, pyridyl, naphthyl, or quinolinyl, including the fused ring when present, is optionally and independently substituted by halo, R, OR, SR, OH, NO₂, CN, NH₂, NHR, N(R)₂, NHCOR, NHCONHR, NHCON(R)₂, NRCOR, NHCO₂R, CO₂R, CO₂H, COR, CONHR, CON(R)₂, S(O)₂R, SONH₂, S(O)R, SO₂NHR, or NHS(O)₂R; and
wherein each substitutable nitrogen atom of said optionally substituted pyridyl or quinolinyl is optionally substituted by R, COR, S(O)₂R, or CO₂R.

As used herein, the following definitions shall apply unless otherwise indicated. The term "aliphatic" as used herein means straight chained, branched or cyclic C₁-C₁₂ hydrocarbons, preferably one to six carbons, which are completely saturated or which contain one or more units of unsaturation but which are not aromatic. For example, suitable aliphatic groups include substituted or unsubstituted linear, branched or cyclic alkyl, alkenyl, alkynyl groups and hybrids thereof such as (cycloalkyl)alkyl, (cycloalkenyl)alkyl or (cycloalkyl)alkenyl. The term "alkyl" and "alkoxy" used alone or as part of a larger moiety refers to both straight and branched chains containing one to twelve carbon atoms. The terms "alkenyl" and "alkynyl" used alone or as part of a larger moiety shall include both straight and branched chains containing two to twelve carbon atoms. The terms "haloalkyl", "haloalkenyl" and "haloalkoxy" means alkyl, alkenyl or alkoxy, as the case may be, substituted with one or more halogen atoms. The term "halogen" means F, Cl, Br, or I. The term "heteroatom" means N, O or S and shall include any oxidized form of nitrogen and sulfur, and the quaternized form of any basic nitrogen.

The term "aryl", used alone or as part of a larger moiety as in "aralkyl", refers to aromatic ring groups having five to fourteen members, such as phenyl, benzyl, 1-naphthyl, 2-naphthyl, 1-anthracyl and 2-anthracyl, and heterocyclic aromatic groups or heteroaryl groups such as 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 3-pyridazinyl, 2-thiazolyl, 4-thiazolyl, 5-thiazolyl, 5-tetrazolyl, 2-triazolyl, 5-triazolyl, 2-thienyl, or 3-thienyl.

Aryl groups also include fused polycyclic aromatic ring systems in which a carbocyclic aromatic ring or heteroaryl ring is fused to one or more other rings. Examples include tetrahydronaphthyl, benzimidazolyl, benzothienyl, benzofuranyl, indolyl, quinolinyl, benzodiazepinyl, benzothiazolyl, benzooxazolyl, benzimidazolyl, isoquinolinyl, isoindolyl, acridinyl, benzoisoxazolyl, and the like. Also included within the scope of the term "aryl", as it is used herein, is a group in which one or more carbocyclic aromatic rings and/or heteroaryl rings are fused to a cycloalkyl or non-aromatic heterocyclyl, for example, indanyl or tetrahydrobenzopyranyl.

The term "heterocyclic ring" or "heterocyclyl" refers to a non-aromatic ring which includes one or more heteroatoms such as nitrogen, oxygen or sulfur in the ring. The ring can be five, six, seven or eight-membered and/or fused to another ring, such as a cycloalkyl or aromatic ring. Examples include 3-1H-benzimidazol-2-one, 3-1-alkyl-benzimidazol-2-one, 2-tetrahydrofuranyl, 3-tetrahydrofuranyl, 2-tetrahydropyranyl, 3-tetrahydropyranyl, 4-tetrahydropyranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, and benzothiane.

A compound of this invention may contain a ring that is fused to a partially saturated or fully unsaturated five to seven membered ring containing zero to three heteroatoms. Such a fused ring may be an aromatic or non-aromatic monocyclic ring, examples of which include the aryl and heterocyclic rings described above.

An aryl group (carbocyclic and heterocyclic) or an aralkyl group, such as benzyl or phenethyl, may contain one or more substituents. Examples of suitable substituents on the unsaturated carbon atom of an aryl group include a halogen, -R, -OR, -OH, -SH, -SR, protected OH (such as acyloxy), phenyl (Ph), substituted Ph, -OPh, substituted -OPh, -NO₂, -CN, -NH₂, -NHR, -N(R)₂, -NHCOR, -NHCONHR, -NHCON(R)₂, -NRCOR, - NHCO₂R, -CO₂R, -CO₂H, -COR, -CONHR, -CON(R)₂, -S(O)₂R, - SONH₂, -S(O)R, -SO₂NHR, or -NHS(O)₂R, where R is an aliphatic group or a substituted aliphatic group.

An aliphatic group or a non-aromatic heterocyclic ring may contain one or more substituents. Examples of suitable substituents on the saturated carbon of an aliphatic group or of a non-aromatic heterocyclic ring include those listed above for the unsaturated carbon, such as in an aromatic ring, as well as the following: =O, =S, =NNHR, =NNR₂, =N-OR, =NNHCOR, =NNHCO₂R, =NNHSO₂R, or =NR.

A substitutable nitrogen on an aromatic or non-aromatic heterocyclic ring may be optionally substituted. Suitable substituents on the nitrogen include R, COR, S(O)₂R, and CO₂R, where R is an aliphatic group or a substituted aliphatic group.

Compounds derived by making isosteric or bioisosteric replacements of carboxylic acid or ester moieties of compounds described herein are within the scope of this invention. Isosteres, which result from the exchange of an atom or group of atoms to create a new compound with similar biological properties to the parent carboxylic acid or ester, are known in the art. The bioisosteric replacement may be physicochemically or topologically based. An example of an isosteric replacement for a carboxylic acid is CONHSO₂(alkyl) such as CONHSO₂Me.

It will be apparent to one skilled in the art that certain compounds of this invention may exist in tautomeric forms or hydrated forms, all such forms of the compounds being within the scope of the invention. Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the invention. Unless otherwise stated, structures depicted herein are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon are within the scope of this invention. Such compounds are useful, for example, as analytical tools or probes in biological assays.

Examples of the R² group include, but are not limited to, hydrogen, methyl, alkoxymethyl (such as methoxymethyl, methoxyethoxymethyl and ethoxymethyl), benzyloxymethyl, and heterocyclylmethyl (such as piperidin-1-ylmethyl, morpholin-4-ylmethyl, and tetrahydrofuran-3-ylmethyl), each of which may be optionally substituted. Examples of the R³ group include, but are not limited to, phenyl and benzyl, each of which may be optionally substituted.

Table 1 below shows examples of compounds of formula II.

| **No.** | **G** | **R¹** | **R²** | **R³** |
|---|---|---|---|---|
| II-1 | CH₃ | Phenyl | H | Ph |
| II-2 | CH₃ | 4-methoxy-phenyl | H | Ph |
| II-3 | CH₃ | 3,4-dimethoxy-phenyl | H | Ph |
| II-4 | CH₃ | 3,5-dimethoxy-phenyl | H | Ph |
| II-5 | CH₃ | 4-cyano-phenyl | H | Ph |
| II-6 | CH₃ | 3-fluoro-phenyl | H | Ph |
| II-7 | CH₃ | 4-fluoro-phenyl | H | Ph |
| II-8 | CH₃ | 4-COCH₃-phenyl | H | Ph |
| II-9 | CH₃ | 4-CONH₂-phenyl | H | Ph |
| II-10 | CH₃ | 4-SCH₃-phenyl | H | Ph |
| II-11 | CH₃ | 3-OCH₃-phenyl | H | Ph |
| II-12 | CH₃ | 3,4,5-trimethoxy-phenyl | H | Ph |
| II-13 | CH₃ | 4-CO₂CH₃-phenyl | H | Ph |
| II-14 | CH₃ | 4-SO₂CH₃-phenyl | H | Ph |
| II-15 | CH₃ | 4-CO₂CH₃-phenyl | H | Ph |
| II-16 | CH₃ | 4-N(CH₃)₂-phenyl | H | Ph |
| II-17 | CH₃ | 3-NO₂-phenyl | H | Ph |
| II-18 | CH₃ | 3-NHCOCH₃-phenyl | H | Ph |
| II-19 | CH₃ | 3-NH₂-phenyl | H | Ph |
| II-20 | CH₃ | 4-NO₂-phenyl | H | Ph |
| II-21 | CH₃ | 3-(CH₂CH₂CO₂H)-phenyl | H | Ph |
| II-22 | CH₃ | 3-(CH₂CO₂H)-phenyl | H | Ph |
| II-23 | CH₃ | 3-CH₂OH-phenyl | H | 4-CH₃-Ph |
| II-24 | CH₃ | Phenyl | H | 4-OMe-phenyl |
| II-25 | CH₃ | 4-methoxy-phenyl | H | 4-OMe-phenyl |
| II-26 | CH₃ | 3,4-dimethoxy-phenyl | H | 4-Cl-Ph |
| II-27 | CH₃ | 3,5-dimethoxy-phenyl | H | 3,4-Cl₂-Ph |
| II-28 | CH₃ | 4-cyano-phenyl | H | 4-F-Ph |
| II-29 | CH₃ | 3-fluoro-phenyl | H | 4-OMe-phenyl |
| II-30 | CH₃ | 4-fluoro-phenyl | H | 2,5-Cl₂-PhPh |
| II-31 | CH₃ | 4-COCH₃-phenyl | H | 2,4-F₂-Ph |
| II-32 | CH₃ | 4-CONH₂-phenyl | H | 4-NO₂-Ph |
| II-33 | CH₃ | 4-SCH₃-phenyl | H | 3,5-Cl₂-Ph |
| II-34 | CH₃ | 3-OCH₃-phenyl | H | 3-Cl-Ph |
| II-35 | CH₃ | 3,4,5-trimethoxy-phenyl | H | 4-OMe-phenyl |
| II-36 | CH₃ | 4-CH₃-phenyl | H | 3-OBn-Ph |
| II-37 | CH₃ | cyclohexyl | H | 4-OMe-phenyl |
| II-38 | CH₃ | cyclohexyl | H | 4-OMe-phenyl |
| II-39 | CH₃ | cyclohexyl | H | 4-Cl-Ph |
| II-40 | CH₃ | cyclohexyl | H | 3,4-Cl₂-Ph |
| II-41 | CH₃ | cyclohexyl | H | 4-F-Ph |
| II-42 | CH₃ | cyclohexyl | H | 4-OMe-phenyl |
| II-43 | CH₃ | cyclohexyl | H | 2,5-Cl₂-Ph |
| II-44 | CH₃ | cyclohexyl | H | 2,4-F₂-Ph |
| II-45 | CH₃ | cyclohexyl | H | 4-NO₂-Ph |
| II-46 | CH₃ | cyclohexyl | H | 3,5-Cl₂-Ph |
| II-47 | CH₃ | cyclohexyl | H | 3-Cl-Ph |
| II-48 | CH₃ | cyclohexyl | H | 4-OMe-phenyl |
| II-49 | CH₃ | cyclohexyl | H | 3-OBn-Ph |
| II-50 | CH₃ | cyclohexyl | H | -CH₂Ph |
| II-51 | CH₃ | cyclohexyl | H | Ph |
| II-52 | CH₃ | Phenyl | H | 4-OMe-phenyl |
| II-53 | H | 4-methoxy-phenyl | H | 4-OMe-phenyl |
| II-54 | H | 3,4-dimethoxy-phenyl | H | 4-Cl-Ph |
| II-55 | H | 3,5-dimethoxy-phenyl | H | 3,4-Cl₂-Ph |
| II-56 | H | 4-cyano-phenyl | H | 4-F-Ph |
| II-57 | H | 3-fluoro-phenyl | H | 4-OMe-phenyl |
| II-58 | H | 4-fluoro-phenyl | H | 2,5-Cl₂-PhPh |
| II-59 | H | 4-COCH₃-phenyl | H | 2,4-F₂-Ph |
| II-60 | H | 4-CONH₂-phenyl | H | 4-NO₂-Ph |
| II-61 | H | 4-SCH₃-phenyl | H | 3,5-Cl₂-Ph |
| II-62 | H | 3-OCH₃-phenyl | H | 3-Cl-Ph |
| II-63 | H | 3,4,5-trimethoxy-phenyl | H | 4-OMe-phonyl |
| II-64 | H | 4-CH₃-phenyl | H | 3-OBn-Ph |
| II-65 | H | cyclohexyl | H | benzyl |
| II-66 | H | cyclohexyl | H | 4-OMe-phenyl |
| II-67 | H | cyclohexyl | H | phenyl |
| II-68 | H | cyclohexyl | H | 3,4-Cl₂-Ph |
| II-69 | H | cyclohexyl | H | 2,4-Cl₂-Ph |
| II-70 | H | cyclohexyl | H | 4-OMe-phenyl |
| II-71 | H | cyclohexyl | H | 2,5-Cl₂-Ph |
| II-72 | H | cyclohexyl | H | 2,4-F₂-Ph |
| II-73 | H | cyclohexyl | H | 4-NO₂-Ph |
| II-74 | H | cyclohexyl | H | 3,5-Cl₂-Ph |
| II-75 | H | cyclohexyl | H | 3-Cl-Ph |
| II-76 | H | Phenyl | H | 4-OMe-phenyl |
| II-78 | CH₃ | 4-methoxy-phenyl | H | -CH₂Ph |
| II-79 | CH₃ | 3,4-dimethoxy-phenyl | H | -CH₂Ph |
| II-80 | CH₃ | 3,5-dimethoxy-phenyl | H | -CH₂Ph |
| II-81 | CH₃ | 4-cyano-phenyl | H | -CH₂Ph |
| II-82 | CH₃ | 3-fluorophenyl | H | -CH₂Ph |
| II-83 | CH₃ | 3,4,5-trimethoxy-phenyl | H | -CH₂Ph |
| II-84 | CH₃ | 3-pyridyl | H | Ph |
| II-85 | CH₃ | 4-methoxy-pyrid-3-yl | H | Ph |
| II-86 | CH₃ | 2-naphthyl | H | Ph |
| II-88 | CH₃ | 6-methoxy-naphthalen-2-yl | H | Ph |
| II-90 | CH₃ | 2-methyl-quinolin-6-yl | H | Ph |
| II-91 | CH₃ | 4-methoxy-phenyl | CH₃ | Ph |
| II-92 | CH₃ | 3,4-dimethoxy-phenyl | CH₃ | Ph |
| II-93 | CH₃ | 3,5-dimethoxy-phenyl | CH₃ | 4-OMe-phenyl |
| II-94 | CH₃ | cyclohexyl | CH₃ | 4-OMe-phenyl |
| II-95 | CH₃ | cyclohexyl | CH₃ | 4-Cl-phenyl |
| II-96 | CH₃ | cyclohexyl | CH₃ | Ph |
| II-97 | CH₃ | 4-mothoxy-phenyl | CH₃ | -CH₂Ph |
| II-98 | CH₃ | 2-methyl-quinolin-6-yl | CH₃ | -CH₂Ph |
| II-99 | CH₃ | 2-methyl-quinolin-6-yl | CH₃ | -CH₂Ph |
| II-100 | H | 4-F-phenyl | CH₃ | Ph |
| II-101 | H | 4-Cl-phenyl | CH₃ | Ph |
| II-102 | H | 4-NO₂-phenyl | CH₃ | Ph |
| II-103 | H | cyclohexyl | CH₃ | 2,6-difluoro-phenyl |
| II-104 | H | cyclohexyl | CH₃ | 3,5-dichloro-phenyl |
| II-105 | H | cyclohexyl | CH₃ | 2,4-dichloro-phenyl |
| II-106 | H | cyclohexyl | CH₃ | Ph |
| II-107 | H | 3-Cl-phenyl | CH₃ | Ph |
| II-108 | H | 3-(benzyloxy)-phenyl | CH₃ | Ph |
| II-109 | H | phenyl | CH₃ | 2,4-difluoro-phenyl |
| II-110 | CH₃ | 3-Cl-phenyl | H | phenyl |
| II-111 | H | phenyl | H | 2,4-difluoro-phenyl |
| II-112 | H | cyclohexyl | H | phenyl |
| II-113 | H | 3-Br-phenyl | CH₃ | phenyl |
| II-114 | H | 3-I-phenyl | CH₃ | phenyl |
| II-115 | H | 2-chloropyridin-5-yl | CH₃ | phenyl |
| II-116 | H | phenyl | CH₃ | pyridin-2-yl |
| II-117 | H | 4-F-phenyl | CH₃ | pyridin-2-yl |
| II-118 | H | 4-Cl-phenyl | CH₃ | pyridin-2-yl |
| II-119 | H | 3-Cl-phenyl | CH₃ | pyridin-2-yl |
| II-120 | H | 4-NO₂-phenyl | CH₃ | pyridin-2-yl |
| II-121 | H | 3-(benzyloxy)-phenyl | CH₃ | pyridin-2-yl |
| II-122 | H | 2,6-difluorophenyl | CH₃ | phenyl |
| II-123 | H | phenyl | CH₃ | 3-Cl-phenyl |
| II-124 | H | 4-F-phenyl | CH₃ | 3-Cl-phenyl |
| II-125 | H | 4-Cl-phenyl | CH₃ | 3-Cl-phenyl |
| II-126 | H | 3-Cl-phenyl | CH₃ | 3-Cl-phenyl |
| II-127 | H | 4-NO₂-phenyl | CH₃ | 3-Cl-phonyl |
| II-128 | H | 3-(benzyloxy)-phenyl | CH₃ | 3-Cl-phenyl |
| II-129 | H | naphthalen-2-yl | CH₃ | phenyl |
| II-130 | H | 3,4-dimethoxyphenyl | CH₃ | phenyl |
| II-131 | H | phenyl | CH₃ | 6-CH₃-4-CF₃-pyridin2-yl |
| II-132 | H | 4-F-phenyl | CH₃ | 6-CH₃-4-CF₃-pyridin2-yl |
| II-133 | H | 4-Cl-phenyl | CH₃ | 6-CH₃-4-CF₃-pyridin2-yl |
| II-134 | H | 3-Cl-phenyl | CH₃ | 6-CH₃-4-CF₃-pyridin2-yl |
| II-135 | H | 4-NO₂-phenyl | CH₃ | 6-CH₃-4-CF₃-pyridin2-yl |
| II-136 | H | 3-(benzyloxy)-phenyl | CH₃ | 6-CH₃-4-CF₃-pyridin2-yl |
| II-137 | H | 3-F-phenyl | CH₃ | pyridin-2-yl |
| II-138 | H | 3-chloro-4-methoxyphenyl | CH₃ | pyridin-2-yl |
| II-139 | H | naphthalen-2-yl | CH₃ | pyridin-2-yl |
| II-141 | H | 3-OH-phenyl | CH₃ | phenyl |
| II-142 | H | 3-(benzyloxy)-phenyl | CH₃ | phenyl |
| II-143 | H | 4-SO₂NH₂-phenyl | CH₃ | phenyl |
| II-144 | H | 4-SO₂NH₂-phenyl | CH₃ | pyridin-2-yl |
| II-145 | H | 3- NH₂-phenyl | CH₃ | phenyl |
| II-146 | CH₂CH₃ | 3-phenoxy-phenyl | CH₃ | phenyl |
| II-147 | CH₂CH₃ | 3-methoxy-phenyl | CH₃ | 4-F-phenyl |
| II-148 | CH₂CH₃ | 3-(benzyloxy)-phenyl | CH₃ | 4-F-phenyl |
| II-149 | CH₂CH₃ | 3-Cl-phenyl | CH₃ | 4-F-phenyl |
| II-150 | CH₂CH₃ | 3-(benzyloxy)-phenyl | CH₃ | 3-NO₂-phenyl |
| II-151 | CH₂CH₃ | 3-methoxy-phenyl | CH₃ | 3-NO₂-phenyl |
| II-152 | CH₂CH₃ | 3-(benzyloxy)-phenyl | CH₃ | 3-CF₃-phenyl |
| II-153 | CH₂CH₃ | 3-phenoxy-phenyl | CH₃ | 3-CF₃-phenyl |
| II-154 | CH₂CH₃ | 3-Cl-phenyl | CH₃ | 3-CF₃-phenyl |
| II-155 | CH₂CH₃ | 3-methoxy-phenyl | CH₃ | 3-CF₃-phenyl |
| II-156 | CH₂CH₃ | 3-(benzyloxy)-phenyl | CH₃ | 4-NO₂-phenyl |
| II-157 | CH₂CH₃ | 3-phenoxy-phenyl | CH₃ | 4-NO₂-phenyl |
| II-158 | CH₂CH₃ | 3-Cl-phenyl | CH₃ | 4-NO₂-phenyl |
| II-159 | CH₂CH₃ | 3-methoxy-phenyl | CH₃ | 4-NO₂-phenyl |

The compounds of this invention may be prepared in general by methods known to those skilled in the art for analogous compounds, as illustrated by the general schemes below and by the preparative examples that follow. 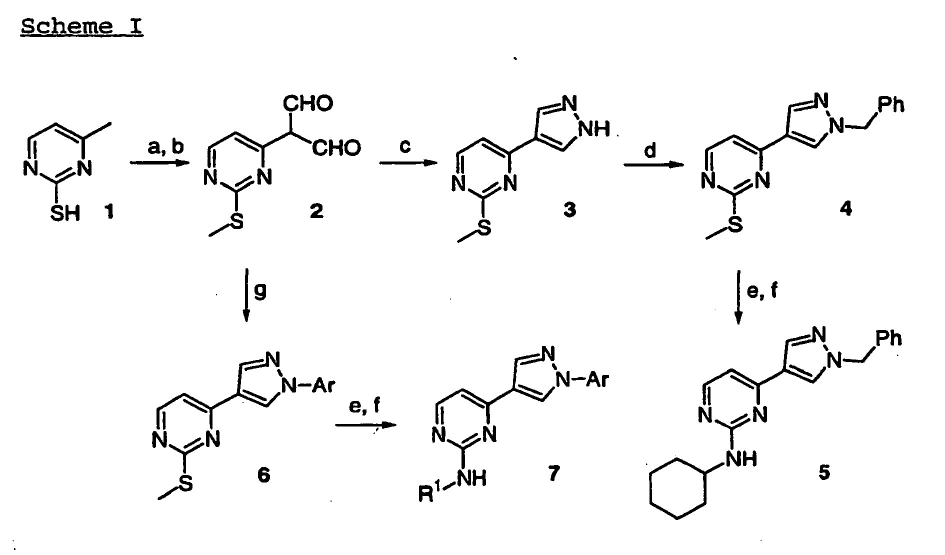 Reagents and conditions: a) MeI, NaOH (aqueous); (b) COCl₂, DMF, CHCl₃; (c) NH₂NH₂, MeOH, 65°C; (d) benzylbromide, CH₃CN, K₂CO₃; (e) Oxone^{®}, MeOH/H₂O (1:1); (f) R¹NH₂, DMSO, 85°C or R¹NH₂ (neat), 100°C; (g) ArNHNH₂, MeOH, 65°C

Scheme I above shows a synthetic scheme that may be used to prepare compounds of the present invention wherein G and R² are each hydrogen. Oxone® is potassium peroxymonosulfate. For illustrative purposes, the steps from compound 3 to prepare compound 5 introduce R¹ as a cyclohexyl group and R³ as a benzyl group. It will be understood to one skilled in the art that other R¹ and R³ groups may be introduced in a similar fashion using other R¹ amines and other bromides of R³ or the like. 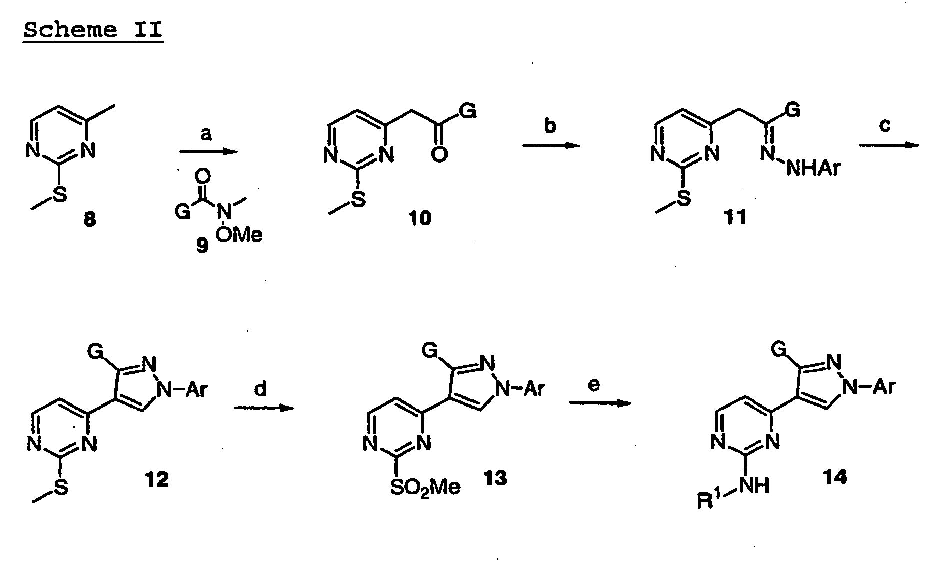 Reagents and conditions: a) i: LDA, THF, -78 °C, ii: **9**, -78°C → 0°C; (b) ArNHNH₂, MeOH, 100°C; (c) DMF-DMA, 100°C; (d) Oxone^{®}, MeOH/H₂O(1:1); (e) R¹NH₂, DMSO, 80°C or R¹NH₂ (neat), 100°C

Scheme II above shows a synthetic scheme that may be used to prepare compounds of the present invention where R² is hydrogen and G is other than hydrogen. 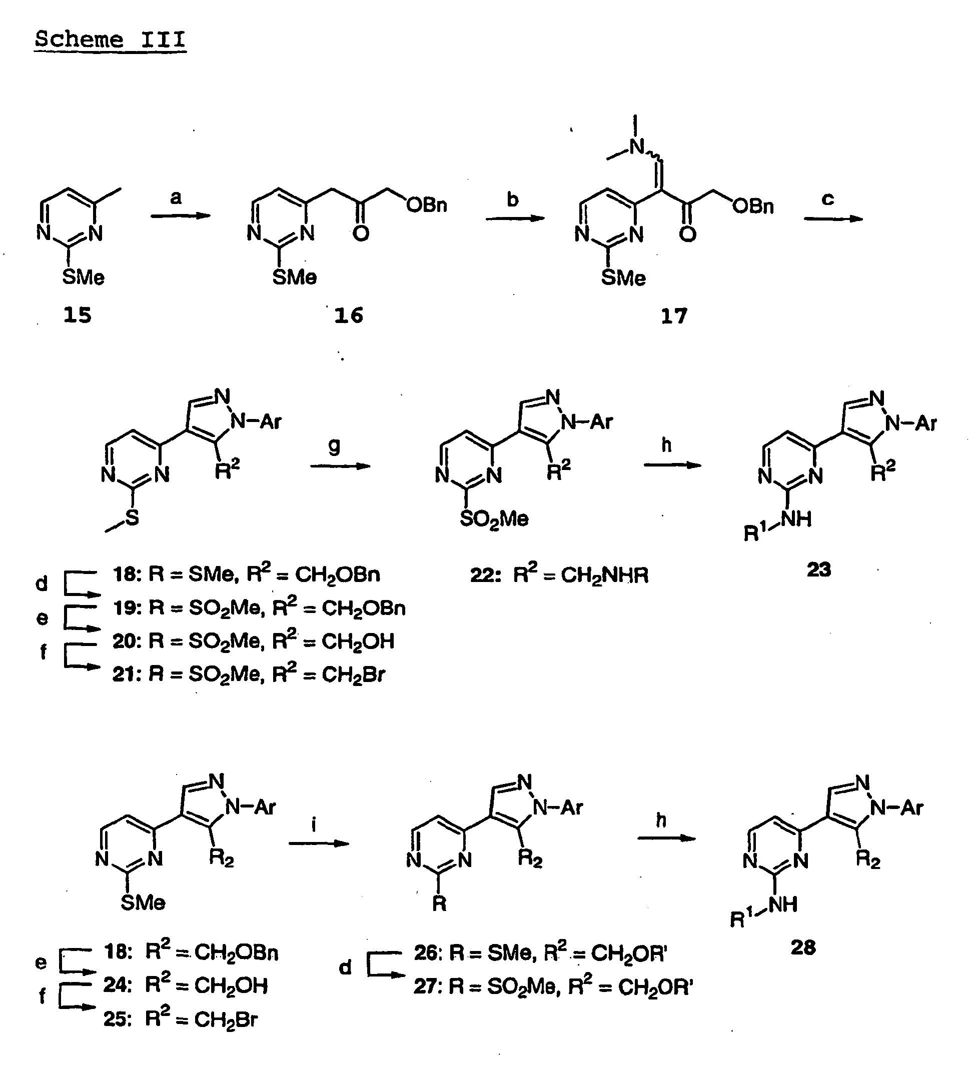 Reagents and conditions: a)i. LDA, THF, -78°C; ii. BnOCH₂CON(OCH₃)CH₃, -78°C → 0°C; (b) DMF-DMA, 50°C; (c)ArNHNH₂, EtOH, 80°C; (d) Oxone®, MeOH/H₂O(1:1); (e) TMSI, CHCl₃; (f) PPh₃, CBr₄, CH₂Cl₂; (g) RNH₂, DMF; (h) R¹NH₂, DMF, 40°C or R¹NH₂ (neat), 100°C; (i)NaH, R'OH, THF

Scheme III above shows a synthetic scheme for preparing compounds of this invention where G is hydrogen and R² is other than hydrogen. While the above schemes describe routes to compounds wherein Q is a pyrimidine ring, it will be apparent to one skilled in the art that compounds where Q is a pyridine ring may be prepared in an analogous manner. 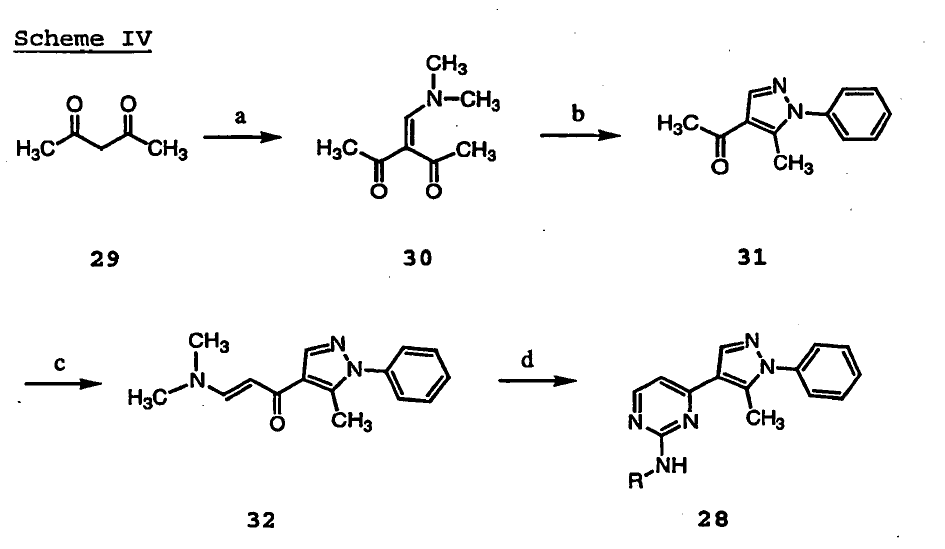 Reagents and conditions: (a) Dimethylformamide dimethyl acetal (DMF-DMA), CH₃CN, 80°C; (b) Ph-NHNH₂, MeOH; (c) Dimethylformamide dimethyl acetal (DMF-DMA), CH₃CN, 80°C, 18h; (d) RNHC(=NH)NH₂, CH₃CN, 80°C, 24h.

Scheme IV above shows an alternative route to the preparation of the present compounds wherein the pyrimidine ring is made at the end of the synthesis (step d). 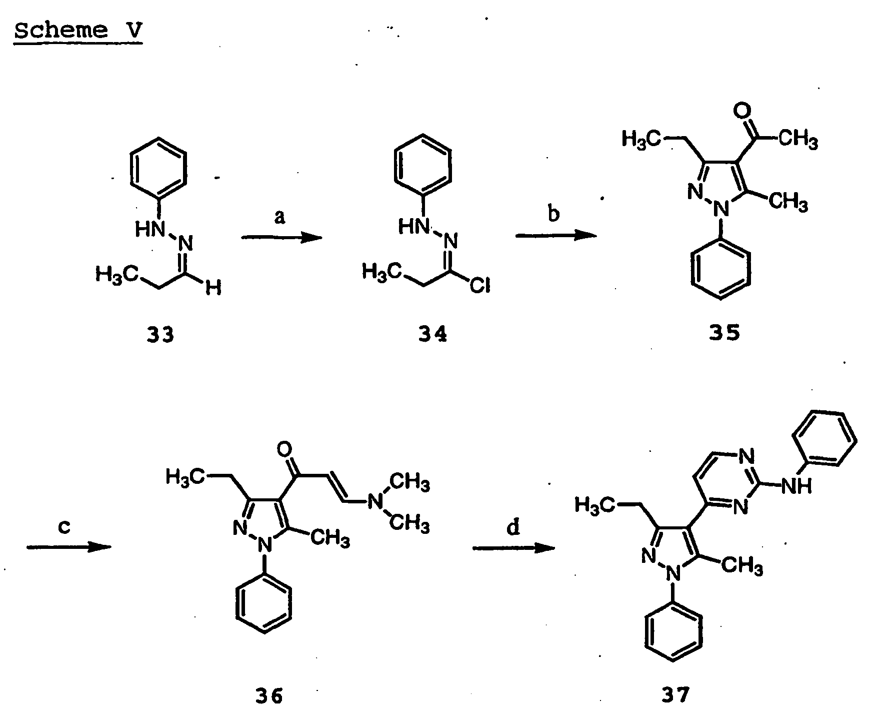 Reagents and conditions: (a) N-chlorosuccinimide, (CH₃)₂S CH₂Cl₂, -78°C; (b) (CH₃CO)₂CH₂, EtOH, NaOH, room temperature; (c) dimethylformamide dimethyl acetal (DMF-DMA), toluene, reflux; (d) PhNHC(=NH)NH₂, NaOMe, MeOH, reflux, 36h (sealed tube).

Scheme V above shows an alternative route for the preparation of the present compounds wherein R² is other than hydrogen. After the reactions and standard work-up conditions, compounds **34** and **36** were used in the next step without purification. Compound **35** was purified by column chromatography and compound **37** was purified by preparative HPLC. While this route is illustrated for compound 37, it will be appreciated by one skilled in the art that this general approach is applicable to the preparation of compounds having other G and R¹-R³ groups.

The compounds of this invention were found to be surprisingly potent inhibitors of all three JNK isoforms (JNK1, JNK2 and JNK3). A significant difference between the present compounds and the most closely related prior art compounds was found to be in the improved inhibition of JNK1 and the selectivity for JNK over p38 activity, as described below.

The activity of the JNK inhibitors of this invention may be assayed *in vitro, in vivo* or in a cell line. In vitro assays include assays that determine inhibition of either the kinase activity or ATPase activity of activated JNK. For example, see the testing examples described below. Alternate *in vitro* assays quantitate the ability of the inhibitor to bind to JNK and may be measured either by radiolabelling the inhibitor prior to binding, isolating the inhibitor/JNK complex and determining the amount of radiolabel bound, or by running a competition experiment where new inhibitors are incubated with JNK bound to known radioligands. One may use any type or isoform of JNK, depending upon which JNK type or isoform is to be inhibited.

The JNK inhibitors or pharmaceutical salts thereof may be formulated into pharmaceutical compositions for administration to animals or humans. These pharmaceutical compositions, which comprise an amount of JNK inhibitor effective to treat or prevent a JNK-mediated condition and a pharmaceutically acceptable carrier, are another embodiment of the present invention.

The term "JNK-mediated condition", as used herein means any disease or other deleterious condition in which JNK is known to play a role. Such conditions include, without limitation, inflammatory diseases, autoimmune diseases, destructive bone disorders, proliferative disorders, cancer, infectious diseases, neurodegenerative diseases, allergies, reperfusion/ischemia in stroke, heart attacks, angiogenic disorders, organ hypoxia, vascular hyperplasia, cardiac hypertrophy, thrombin-induced platelet aggregation, and conditions associated with prostaglandin endoperoxidase synthase-2.

Inflammatory diseases which may be treated or prevented by the compounds of this invention include, but are not limited to, acute pancreatitis, chronic pancreatitis, asthma, allergies, and adult respiratory distress syndrome.

Autoimmune diseases which may be treated or prevented by the compounds of this invention include, but are not limited to, glomerulonephritis, rheumatoid arthritis, systemic lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, or graft vs. host disease.

Destructive bone disorders which may be treated or prevented by the compounds of this invention include, but are not limited to, osteoporosis, osteoarthritis and multiple myeloma-related bone disorder.

Proliferative diseases which may be treated or prevented by the compounds of this invention include, but are not limited to, acute myelogenous leukemia, chronic myelogenous leukemia, metastatic melanoma, Kaposi's sarcoma, multiple myeloma and HTLV-1 mediated tumorigenesis.

Angiogenic disorders which may be treated or prevented by the compounds of this invention include solid tumors, ocular neovasculization, infantile haemangiomas. Infectious diseases which may be treated or prevented by the compounds of this invention include, but are not limited to, sepsis, septic shock, and Shigellosis.

Viral diseases which may be treated or prevented by the compounds of this invention include, but are not limited to, acute hepatitis infection (including hepatitis A, hepatitis B and hepatitis C), HIV infection and CMV retinitis.

Neurodegenerative diseases which may be treated or prevented by the compounds of this invention include, but are not limited to, Alzheimer's disease, Parkinson's disease, amyotrophic lateral sclerosis (ALS), epilepsy, seizures, Huntington's disease, traumatic brain injury, ischemic and hemorrhaging stroke, cerebral ischemias or neurodegenerative disease, including apoptosis-driven neurodegenerative disease, caused by traumatic injury, acute hypoxia, ischemia or glutamate neurotoxicity.

"JNK-mediated conditions" also include ischemia/reperfusion in stroke, heart attacks, myocardial ischemia, organ hypoxia, vascular hyperplasia, cardiac hypertrophy, hepatic ischemia, liver disease, congestive heart failure, pathologic immune responses such as that caused by T cell activation and thrombin-induced platelet aggregation.

In addition, JNK inhibitors of the instant invention may be capable of inhibiting the expression of inducible pro-inflammatory proteins. Therefore, other "JNK-mediated conditions" which may be treated by the compounds of this invention include edema, analgesia, fever and pain, such as neuromuscular pain, headache, cancer pain, dental pain and arthritis pain.

The compounds of this invention are also useful as inhibitors of Src-family kinases, especially Src and Lck. For a general review of these kinases see Thomas and Brugge, Annu. Rev. Cell Dev. Biol. (1997) 13, 513; Lawrence and Niu, Pharmacol. Ther. (1998) 77, 81; Tatosyan and Mizenina, Biochemistry (Moscow) (2000) 65, 49. Accordingly, these compounds are useful for treating diseases or conditions that are known to be affected by the activity of one or more Src-family kinases. Such diseases or conditions include hypercalcemia, restenosis, hypercalcemia, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obtructive pulmonary disorder, contact dermatitis, cancer, Paget's disease, asthma, ischemic or reperfusion injury, allergic disease, atopic dermatitis, and allergic rhinitis. Diseases that are affected by Src activity, in particular, include hypercalcemia, osteoporosis, osteoarthritis, cancer, symptomatic treatment of bone metastasis, and Paget's disease. Diseases that are affected by Lck activity, in particular, include autoimmune diseases, allergies, rheumatoid arthritis, and leukemia. Compounds of formula II wherein R¹ is aryl are especially useful for treating diseases associated with the Src-family kinases, particularly Src or Lck.

In addition to the compounds of this invention, pharmaceutically acceptable salts of the compounds of this invention may also be employed in compositions to treat or prevent the above-identified disorders.

A "pharmaceutically acceptable salt" of a compound of this invention, means any pharmaceutically acceptable salt, which, upon administration to a recipient, is capable of providing, either directly or indirectly, a compound of this invention or an inhibitorily active metabolite or residue thereof. Particularly favored salts are those that increase the bioavailability of the compounds of this invention when such compounds are administered to a mammal (e.g., by allowing an orally administered compound to be more readily absorbed into the blood) or which enhance delivery of the parent compound to a biological compartment (e.g., the brain or lymphatic system) relative to the parent species.

Pharmaceutically acceptable salts of the compounds of this invention include, without limitation, esters, amino acid esters, phosphate esters, metal salts and sulfonate esters.

Pharmaceutically acceptable salts of the compounds of this invention include those derived from pharmaceutically acceptable inorganic and organic acids and bases. Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-naphthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecanoate. Other acids, such as oxalic, while not in themselves pharmaceutically acceptable, may be employed in the preparation of salts useful as intermediates in obtaining the compounds of the invention and their pharmaceutically acceptable acid addition salts.

Salts derived from appropriate bases include alkali metal (e.g., sodium and potassium), alkaline earth metal (e.g., magnesium), ammonium and N⁺(C₁₋₄ alkyl)₄ salts. This invention also envisions the quaternization of any basic nitrogen-containing groups of the compounds disclosed herein. Water or oil-soluble or dispersible products may be obtained by such quaternization.

Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously.

Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a nontoxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutical compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the pharmaceutical compositions of this invention may be administered in the form of suppositories for rectal administration. These can be prepared by mixing the agent with a suitable non-irritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The amount of JNK inhibitor that may be combined with the carrier materials to produce a single dosage form will vary depending upon the host treated, the particular mode of administration. Preferably, the compositions should be formulated so that a dosage of between 0.01 - 100 mg/kg body weight/day of the inhibitor can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of inhibitor will also depend upon the particular compound in the composition.

According to another embodiment, the invention provides methods for treating or preventing a JNK-mediated condition comprising the step of administering to a patient one of the above-described pharmaceutical compositions. The term "patient", as used herein, means an animal, preferably a human.

Preferably, that method is used to treat or prevent a condition selected from inflammatory diseases, autoimmune diseases, destructive bone disorders, proliferative disorders, infectious diseases, degenerative diseases, neurodegenerative diseases, allergies, reperfusion/ischemia in stroke, heart attacks, angiogenic disorders, organ hypoxia, vascular hyperplasia, cardiac hypertrophy, and thrombin-induced platelet aggregation, or any specific disease or disorder described above.

Depending upon the particular JNK-mediated condition to be treated or prevented, additional drugs, which are normally administered to treat or prevent that condition, may be administered together with the inhibitors of this invention. For example, chemotherapeutic agents or other anti-proliferative agents may be combined with the JNK inhibitors of this invention to treat proliferative diseases.

Those additional agents may be administered separately, as part of a multiple dosage regimen, from the JNK inhibitor-containing composition. Alternatively, those agents may be part of a single dosage form, mixed together with the JNK inhibitor in a single composition.

In order that the invention described herein may be more fully understood, the following examples are set forth. It should be understood that these examples are for illustrative purposes only and are not to be construed as limiting this invention in any manner.

### Example 1

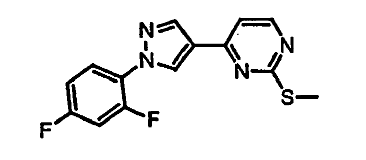

**4-[1-(2,4-difluoro-phenyl)-1*H*-pyrazol-4-yl]-2-methanesulfanyl-pyrimidine.** A 25 mL round-bottom flask equipped with a stirbar was charged with 400 mg (2.04 mmol, 1.00 equivalents) of 2-(2-methanesulfanyl-pyrimidin-4-yl)-malonaldehyde (obtained according to Brown et al., Aust. J. Chem. 33 (10), 2291-2298, 1980), 400 mg of 2,4-difluorophenylhydrazine (2.78 mmol, 1.36 equiv.) and 10 mL of methanol. The resulting solution was stirred at 65 °C for 12 h and then concentrated to a yellow oil which was purified by silica gel chromatography (CH₂Cl₂, 15 x 1 cm) to provide 315 mg (1.04 mmol, 50.7% yield) of the product as a yellow foam.

### Example 2

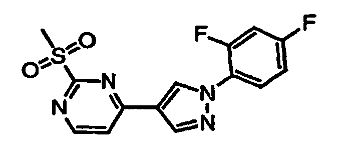

**4-[1-(2,4-difluoro-phenyl)-1*H*-pyrazol-4-yl]-2-methanesulfonyl-pyrimidine.** To a stirred solution of 315 mg of 4-[1-(2,4-difluoro-phenyl)-1*H*-pyrazol-4-yl]-2-methanesulfanyl-pyrimidine (1.04 mmol, 1 equiv) in 20 mL of a 1:1 mixture of methanol and water (v:v) was added 500 mg of Oxone^{®} as a single portion. The reaction mixture was allowed to stir 12 h at 25 °C and then partitioned between CH₂Cl₂ and water. The water layer was washed twice with CH₂Cl₂ and the orgainc layers were combined and dried over anhydrous Na₂SO₄. The solution was concentrated to give a yellow solid which was used without further purification. ¹H NMR (500 MHz, CDCl₃) δ 8.805 (1 H, d, *J* = 5 Hz, Ar), 8.66 (1 H, d, *J* = 3 Hz, Ar), 8.31 (1 H, s, Ar), 7.90-7.89 (1 H, m, Ar), 7.62 (1 H, d, *J* = 5 Hz, Ar), 7.07-7.03 (2 H, m, Ar), 3.39 (3 H, s, SCH₃) ppm.

### Example 3

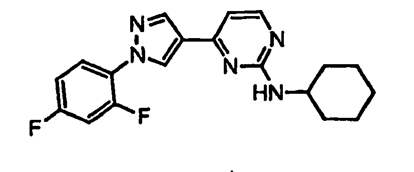

**Cyclohexyl-{4-[1-(2, 4-difluoro-phenyl)-1*H*-pyrazol-4-yl]-pyrimidin-2-yl}-amine.** A 25 mL round-bottom flask equipped with a stirbar was charged with 265 mg (0.787 mmol, 1 equiv) of 4-[1-(2,4- difluoro-phenyl)-1*H-*pyrazol-4-yl]-2-methanesulfonyl-pyrimidine dissolved in 5 mL of DMSO. To the stirred solution was added 100 µL of cyclohexylamine (86.7 mg, 0.874 mmol, 1.10 equiv). The resulting solution was warmed to 85 °C and stirred for 12 h. The orange solution was then partitioned between CH₂Cl₂ and water. The aqueous layer was extracted twice with 20 mL of CH₂Cl₂ and the organic layers were combined, dried over Na₂SO₄ and concentrated to yellow oil, which was purified by silica gel chromatography (1:1 CH₂Cl₂:EtOAc, R_{f} = 0.2) to yield 35 mg (0.098 mmol, 12.5% yield) of product as a foam . ¹H NMR (500 MHz, CDCl₃) δ 8.37 (1 H, d, *J* = 3 Hz, Ar), 8.17 (1 H, d, *J* = 5 Hz, Ar), 8.11 (1 H, s, Ar), 7.83-7.79 (1 H, m, Ar), 6.97-6.94 (2 H, m, Ar), 6.64 (1 H, *J* = 5 Hz, Ar), 5.04 (1 H, br s, NH), 3.82-3.80 (1 H, m, cy-Hx), 2.01-1.97 (3 H, m, cy-Hx), 1.70-1.67 (2H, m, cy-Hx), 1.59-1.57 (1 H, m, cy-Hx), 1.37-1.35 (2 H, m, cy-Hx), 1.20-1.16 (2 H, m, cy-Hx) ppm. MS (M+H): 356.15.

### Example 4

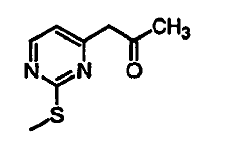

**1-(2-Methylsulfanyl-pyrimidia-4-yl)-propan-2-one.** To a stirred solution of 4-methyl-2-(methylthio)pyrmidine (5.23 g, 38.0 mmol) and phenanthroline (10 mg) in THF (100 mL) at -78 °C was added dropwise a solution of lithium diisopropylamine (1.5 M in cyclohexane) until all water was destroyed and the solution turned dark (∼1 mL). Then, additional lithium diisopropylamine (30.4 mL) was added slowly over 5 min. After 15 min. at -78 °C, the solution was allowed to warm to 0 °C and after 15 min recooled to -78 °C. Then, a solution of N-methoxy-N-methyl-acetamide (4.72 mL, 45.6 mmol) in THF (15 mL), which was dried over 4 molecular sieves for 1 h, was added dropwise via dropfunnel over 20 min. After 25 min. at -78 °C, the solution was warmed to 0 °C and stirred for 30 min. The solution was poured into dilute HCl (1 M, 100 mL), extracted with Et₂O (3 × 200 mL), dried (MgSO₄), filtered and concentrated. Flash chromatography (SiO₂, 20% EtOAc-hexanes) provided the product (4.75 g, 26.1 mmol, 69% yield).

### Example 5

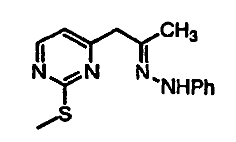

**N-[1-Methyl-2-(2-methylsulfanyl-pyrimidin-4-yl)-ethylidene]-N'-phenyl-hydrazine** A stirred solution of 1-(2-methylsulfanyl-pyrimidin-4-yl)-propan-2-one (182 mg, 1.00 mmol) and phenylhydrazine (99 µL, 1.00 mmol) in dry EtOH (2 mL) in a sealed tube was heated to 100 °C for 15 min. The reaction was concentrated in vacuo to yield the crude product as a yellow paste. 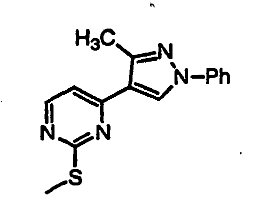 **4-3-Methyl-1-phenyl-1H-pyrazol-4-yl)-2-methylsulfanyl-pyrimidine.** To the crude paste prepared above was added dimethylformamdide-dimethoxyacetal (DMF-DMA, 1.33 mL, 10 mmol). The resulting solution was heated to 100 °C in a sealed tube for three days. The solution was cooled and poured into H₂O (15 mL) and extracted with EtOAc (3 × 15 mL), The combined organic extracts was washed with saturated NH₄Cl (15 mL), brine (15 mL), dried (MgSO₄), filtered and concentrated. Flash chromatography (SiO₂, 30% EtOAc-hexanes) provided the product (225 mg, 0.800 mmol, 80%).

### Example 6

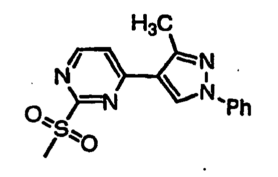

**2-Methanesulfonyl-4-(3-methyl-1-phenyl-1H-pyrazol-4-yl)-pyrimidine** To a stirred solution of 4-(3-Methyl-1-phenyl-1H-pyrazol-4-yl)-2-methylsulfanyl-pyrimidine (225 mg, 0.800 mmol) in MeOH (8 mL) at ambient temperature was added dropwise a solution Oxone^{®} (1.48 g, 2.40 mmol) in H₂O (8 mL) over 20 min. After 16 h., the solution was poured into H₂O (50 mL) and extracted with CH₂Cl₂ (4 × 25 mL). The combined extracts were dried (MgSO₄), filtered and concentrated to provide the product (239 mg, 0.760 mmol, 95%).

### Example 7

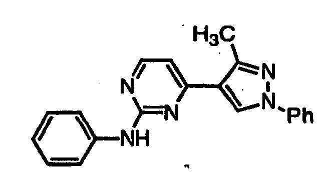

**[4-(3-methyl-1-phenyl-1H-pyrazol-4-yl)-pyrimidin-2-yl]-phenylamine** A mixture of 2-methanesulfonyl-4-(3-methyl-1-phenyl-1H-pyrazol-4-yl)-pyrimidine (38 mg, 0.12 mmol) and aniline (neat, 100 µL) was heated to 100 °C for 15h. The volatile components were evaporated under a stream of nitrogen upon warming. Recrystalization from methanol provided the product (23 mg, 0.070 mmol) in 58% yield.

### Example 8

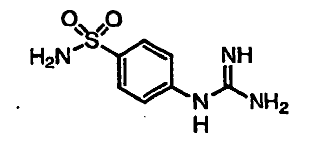

**4-guanidino-benzenesulfonamide** 4-Aminobenzenesulfonamide (30mmol, 1 equiv.), cyanamide (1.3g, 31mmol, 1.03 equiv.), and 4N hydrogen chloride in dioxane (8mL, 32mmol) was stirred for 10 minutes at room temperatura and heated to 80 C for 18 hours. The mixture was diluted with water (30mL) and diethyl ether (50mL). The aqueous layer was washed with ether (30mL) and the organic lalyers were discarded. The aqueous layer was neutralized with 6N aqueous HCl (6mL) and diluted with ethyl acetate (50mL). The aqueous layer was extracted with ethyl acetate (50mL) four times. The combined organic layers were concentrated under reduced pressure to afford a solid compound that was washed with diethyl ether (30mL) to afford pale yellow title compound. The compound was characterized by LC/MS and HPLC.

### Example 9

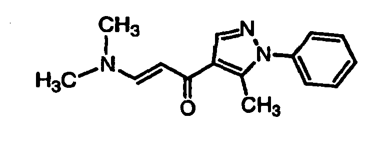

**3-Dimethylamino-1-(5-methyl-1-phenyl-1*H*-pyrazol-4-yl)-propenone** 1-(5-Methyl-1-phenyl-1*H*-pyrazol-4-yl)-ethanone (10mmol) was dissolved in acetonitrile (5mL) and DMF-DMA (2mL) and refluxed for 18 hours. Solvent was removed and trituated with ether (20mL) and the yellow solid was filtered and washed with ether (10mL) to provide the title compound.

### Example 10

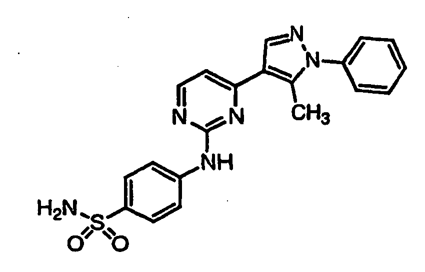

**4-[4-(5-Methyl-1-phenyl-1*H*-pyrazol-4-yl)-pyrimidin-2-ylamino]-benzenesulfonamide** 3-Dimethylamino-1-(5-methyl-1-phenyl-1*H*-pyrazol-4-yl)-propenone (0.2mmol) and 4-guanidino-benzenesulfonamide (0.2mmol) in acetonitrile (0.25mL) was heated to reflux for 24 hours and the resulting mixture was diluted with methanol (3mL). The slurry was filtered and the solid was washed with methanol (2mL) and dried under nitrogen to provide the title compound. M+H (407.0); HPLC Rt = 3.168. HPLC analysis was performed with an HP1100 HPLC and the eight minute analysis method was performed as follows: (0 min, 10%B; 1 min, 30%B; 5 min, 90%B; 6.5min, 90%B; 6.7min 10%B), where A is 0.01% TFA in water and B is 0.01% TFA in acetonitrile. The analytical column was YMC ODS-AQ, 30x100mm with 1mL/min flow rate using a diodo ray detector (5 wavelenths monitored at 300, 280, 254, 220, and 210nm) at 25°C.

### Cloning, Expression and Purification of JNK3 Protein

A BLAST search of the EST database using the published JNK3α1 cDNA as a query identified an EST clone (#632588) that contained the entire coding sequence for human JNK3α1. Polymerase chain reactions (PCR) using pfu polymerase (Strategene) were used to introduce restriction sites into the cDNA for cloning into the pET-15B expression vector at the NcoI and BamHI sites. The protein was expressed in E. coli. Due to the poor solubility of the expressed full-length protein (Met 1-Gln 422), an N-terminally truncated protein starting at Ser residue at position 40 (Ser 40) was produced. This truncation corresponds to Ser 2 of JNK1 and JNK2 proteins, and is preceded by a methionine (initiation) and a glycine residue. The glycine residue was added in order to introduce an NcoI site for cloning into the expression vector. In addition, systematic C-terminal truncations were performed by PCR to identify a construct that give rise to diffraction-quality crystals. One such construct encodes amino acid residues Ser40-Glu402 of JNK3α1 and is preceded by Met and Gly residues.

The construct was prepared by PCR using deoxyoligonucleotides:
5' GCTCTAGAGCTCCATGGGCAGCAAAAGCAAAGTTGACAA 3' (forward primer with initiation codon underlined) (SEQ ID NO:1) and
5' TAGCGGATCCTCATTCTGAATTCATTACTTCCTTGTA 3' (reverse primer with stop codon underlined) (SEQ ID NO:2) as primers and was confirmed by DNA sequencing. Control experiments indicated that the truncated JNK3 protein had an equivalent kinase activity towards myelin basic protein when activated with an upstream kinase MKK7 in vitro.

*E*.*coli* strain BL21 (DE3) (Novagen) was transformed with the JNK3 expression construct and grown at 30°C in LB supplemented with 100 µg/ml carbenicillin in shaker flasks until the cells were in log phase (OD₆₀₀ ∼ 0.8). Isopropylthio-β-D-galactosidase (IPTG) was added to a final concentration of 0.8 mM and the cells were harvested 2 hours later by centrifugation.

*E*. *coli* cell paste containing JNK3 was resuspended in 10 volumes/g lysis buffer (50 mM HEPES, pH 7.2, containing 10% glycerol (v/v), 100 mM NaCl, 2 mM DTT, 0.1 mM PMSF, 2 µg/ml Pepstatin, 1µg/ml each of E-64 and Leupeptin). Cells were lysed on ice using a microfluidizer and centrifuged at 100,000 x *g* for 30 min at 4 °C. The 100,000 x *g* supernatant was diluted 1:5 with Buffer A (20 mM HEPES, pH 7.0, 10% glycerol (v/v), 2 mM DTT) and purified by SP-Sepharose (Pharmacia) cation-exchange chromatography (column dimensions: 2.6 x 20 cm) at 4 °C. The resin was washed with 5 column volumes of Buffer A, followed by 5 column volumes of Buffer A containing 50 mM NaCl. Bound JNK3 was eluted with a 7.5 column volume linear gradient of 50-300 mM NaCl. JNK3 eluted between 150-200 mM NaCl.

### Activation of JNK3

Five mg of JNK3 was diluted to 0.5 mg/ml in 50 mM HEPES buffer, pH 7.5, containing 100 mM NaCl, 5 mM DTT, 20 mM MgCl₂ and 1 mM ATP. GST-MKK7(DD) was added at a molar ratio of 1:2.5 GST-MKK7:JNK3. After incubation for 30 minutes at 25°C, the reaction mixture was concentrated 5-fold by ultrafiltration in a Centriprep-30 (Amicon, Beverly, MA), diluted to 10 ml and an additional 1 mM ATP added. This procedure was repeated three times to remove ADP and replenish ATP. The final addition of ATP was 5 mM and the mixture incubated overnight at 4°C.

The activated JNK3/GST-MKK7(DD) reaction mixture was exchanged into 50 mM HEPES buffer, pH 7.5, containing 5 mM DTT and 5% glycerol (w/v) by dialysis or ultrafiltration. The reaction mixture was adjusted to 1.1 M potassium phosphate, pH 7.5, and purified by hydrophobic interaction chromatography (at 25 °C) using a Rainin Hydropore column. GST-MKK7 and unactivated JNK3 do not bind under these conditions such that when a 1.1 to 0.05 M potassium phosphate gradient is developed over 60 minutes at a flow rate of 1 ml/minute, doubly phosphorylated JNK3 is separated from singly phosphorylated JNK. Activated JNK3 (i.e. doubly phosphorylated JNK3) was stored at -70°C at 0.25-1 mg/ml.

### JNK Inhibition Assays

Compounds were assayed for the inhibition of JNK3 by a spectrophotometric coupled-enzyme assay. In this assay, a fixed concentration of activated JNK3 (10 nM) was incubated with various concentrations of a potential inhibitor dissolved in DMSO for 10 minutes at 30°C in a buffer containing 0.1 M HEPES buffer, pH 7.5, containing 10 mM MgCl₂, 2.5 mM phosphoenolpyruvate, 200 µM NADH, 30 µg/mL pyruvate kinase, 10 µg/mL lactate dehydrogenase, and 200 µM EGF receptor peptide. The EGF receptor peptide has the sequence KRELVEPLTPSGEAPNQALLR, and is a phosphoryl acceptor in the JNK3-catalyzed kinase reaction. The reaction was initiated by the addition of 10 µM ATP and the assay plate is inserted into the spectrophotometer's assay plate compartment that was maintained at 30°C. The decrease of absorbance at 340 nm was monitored as a function of time. The rate data as a function of inhibitor concentration was fitted to competitive inhibition kinetic model to determine the Ki.

The JNK1 and JNK2 assays were performed in an analogous manner.

### Cloning of p38 Kinase in Insect Cells

Two splice variants of human p38 kinase, CSBP1 and CSBP2, have been identified. Specific oligonucleotide primers were used to amplify the coding region of CSBP2 cDNA using a HeLa cell library (Stratagene) as a template. The polymerase chain reaction product was cloned into the pET-15b vector (Novagen). The baculovirus transfer vector, pVL-(His)6-p38 was constructed by subcloning a XbaI-BamHI fragment of pET15b-(His)6-p38 into the complementary sites in plasmid pVL1392 (Pharmingen).

The plasmid pVL-(His)6-p38 directed the synthesis of a recombinant protein consisting of a 23-residue peptide (MGSSHHHHHHSSGLVPRGSHMLE, where LVPRGS represents a thrombin cleavage site) fused in frame to the N-terminus of p38, as confirmed by DNA sequencing and by N-terminal sequencing of the expressed protein. Monolayer culture of Spodoptera frugiperda (Sf9) insect cells (ATCC) was maintained in TNM-FH medium (Gibco BRL) supplemented with 10% fetal bovine serum in a T-flask at 27°C. Sf9 cells in log phase were co-transfected with linear viral DNA of Autographa califonica nuclear polyhedrosis virus (Pharmingen) and transfer vector pVL-(His)6-p38 using Lipofectin (Invitrogen). The individual recombinant baculovirus clones were purified by plaque assay using 1% low melting agarose.

### Expression and Purification of Recombinant p38 Kinase

Trichoplusia ni (Tn-368) High-Five^{™} cells (Invitrogen) were grown in suspension in Excel-405 protein free medium (JRH Bioscience) in a shaker flask at 27°C. Cells at a density of 1.5 X 10⁶ cells/ml were infected with the recombinant baculovirus described above at a multiplicity of infection of 5. The expression level of recombinant p38 was monitored by immunoblotting using a rabbit anti-p38 antibody (Santa Cruz Biotechnology). The cell mass was harvested 72 hours after infection when the expression level of p38 reached its maximum.

Frozen cell paste from cells expressing the (His)₆-tagged p38 was thawed in 5 volumes of Buffer A (50 mM NaH₂PO₄ pH 8.0, 200 mM NaCl, 2mM β-Mercaptoethanol, 10% Glycerol and 0.2 mM PMSF). After mechanical disruption of the cells in a microfluidizer, the lysate was centrifuged at 30,000 x g for 30 minutes. The supernatant was incubated batchwise for 3-5 hours at 4°C with Talon^{™} (Clontech) metal affinity resin at a ratio of 1 ml of resin per 2-4 mgs of expected p38. The resin was settled by centrifugation at 500 x g for 5 minutes and gently washed batchwise with Buffer A. The resin was slurried and poured into a column (approx. 2.6 x 5.0 cm) and washed with Buffer A + 5 mM imidazole.

The (His)₆-p38 was eluted with Buffer A + 100 mM imidazole and subsequently dialyzed overnight at 4°C against 2 liters of Buffer B, (50 mM HEPES, pH 7.5, 25 mM β-glycerophosphate, 5% glycerol, 2mM DTT). The His₆ tag was removed by addition of at 1.5 units thrombin (Calbiochem) per mg of p38 and incubation at 20°C for 2-3 hours. The thrombin was quenched by addition of 0.2 mM PMSF and then the entire sample was loaded onto a 2 ml benzamidine agarose (American International Chemical) column.

The flow through fraction was directly loaded onto a 2.6 x 5.0 cm Q-Sepharose (Pharmacia) column previously equilibrated in Buffer B + 0.2 mM PMSF. The p38 was eluted with a 20 column volume linear gradient to 0.6M NaCl in Buffer B. The eluted protein peak was pooled and dialyzed overnight at 4°C vs. Buffer C (50 mM HEPES pH 7.5, 5% glycerol, 50 mM NaCl, 2 mM DTT, 0.2 mM PMSF).

The dialyzed protein was concentrated in a Centriprep (Amicon) to 3-4 ml and applied to a 2.6 x 100 cm Sephacryl S-100HR (Pharmacia) column. The protein was eluted at a flow rate of 35 ml/hr. The main peak was pooled, adjusted to 20 mM DTT, concentrated to 10-80 mgs/ml and frozen in aliquots at -70°C or used immediately.

### Activation of p38

p38 was activated by combining 0.5 mg/ml p38 with 0.005 mg/ml DD-double mutant MKK6 in Buffer B + 10mM MgCl2, 2mM ATP, 0.2mM Na₂VO₄ for 30 minutes at 20°C. The activation mixture was then loaded onto a 1.0 x 10 cm MonoQ column (Pharmacia) and eluted with a linear 20 column volume gradient to 1.0 M NaCl in Buffer B. The activated p38 eluted after the ADP and ATP. The activated p38 peak was pooled and dialyzed against buffer B + 0.2mM Na₂VO₄ to remove the NaCl. The dialyzed protein was adjusted to 1.1M potassium phosphate by addition of a 4.0M stock solution and loaded onto a 1.0 x 10 cm HIC (Rainin Hydropore) column previously equilibrated in Buffer D (10% glycerol, 20mM β-glycerophosphate, 2.0mM DTT) + 1.1MK₂HPO₄. The protein was eluted with a 20 column volume linear gradient to Buffer D + 50mM K2HPO4. The double phosphorylated p38 eluted as the main peak and was pooled for dialysis against Buffer B + 0.2mM Na₂VO₄ . The activated p38 was stored at -70°C.

### p38 Inhibition Assays

### A. Inhibition of Phosphorylation of EGF Receptor Peptide

This assay is carried out in the presence of 10 mM MgCl₂ and 100 mM HEPES buffer at pH 7.6. For a typical IC₅₀ determination, a stock solution is prepared containing all of the above components and activated p38 (5 nM). The stock solution is aliquotted into vials. A fixed volume of DMSO or inhibitor in DMSO (final concentration of DMSO in reaction is 5%) is introduced to each vial, mixed and incubated for 15 minutes at room temperature. EGF receptor peptide, KRELVEPLTPSGEAPNQALLR, a phosphoryl acceptor in p38-catalyzed kinase reaction, is added to each vial to a final concentration of 200 µM. The kinase reaction is initiated with ATP (100 µM) and the vials are incubated at 30°C. After 30 minutes, the reactions are quenched with equal volume of 10% trifluoroacetic acid (TFA).

The phosphorylated peptide is quantified by HPLC analysis. Separation of phosphorylated peptide from the unphosphorylated peptide is achieved on a reverse phase column (Deltapak, 5 µm, C18 100D, part no. 011795) with a binary gradient of water and acteonitrile, each containing 0.1% TFA. IC₅₀ (concentration of inhibitor yielding 50% inhibition) is determined by plotting the % activity remaining against inhibitor concentration.

### B. Inhibition of ATPase Activity

This assay is carried out in the presence of 10 mM MgCl₂, 25 mM β-glycerophosphate, 10% glycerol and 100 mM HEPES buffer at pH 7.6. For a typical Ki determination, the Km for ATP in the ATPase activity of activated p38 reaction is determined in the absence of inhibitor and in the presence of two concentrations of inhibitor. Ki is determined from the rate data as a function of inhibitor and ATP concentrations. A stock solution is prepared containing all of the above components and activated p38 (60 nM). The stock solution is aliquotted into vials. A fixed volume of DMSO or inhibitor in DMSO (final concentration of DMSO in reaction is 2.5%) is introduced to each vial, mixed and incubated for 15 minutes at room temperature. The reaction is initiated by adding various concentrations of ATP and then incubated at 30°C. After 30 minutes, the reactions are quenched with 50 µl of EDTA (0.1 M, final concentration), pH 8.0. The product of p38 ATPase activity, ADP, is quantified by HPLC analysis.

Separation of ADP from ATP is achieved on a reversed phase column (Supelcosil, LC-18, 3 µm, part no. 5-8985) using a binary solvent gradient of following composition: Solvent A - 0.1 M phosphate buffer containing 8 mM tetrabutylammonium hydrogen sulfate (Sigma Chemical Co., catalogue no. T-7158), Solvent B - Solvent A with 30% methanol.

Table 2 shows the activities of selected compounds in the JNK and p38 inhibition assays. Compounds having a Kᵢ less than 2.0 micromolar are rated "A", compounds having a Kᵢ between 2.0 and 10 micromolar are rated "B" and compounds having a Kᵢ greater than 10 micromolar are rated "C". The last column shows a ratio of p38 to JNK3 activity as a measure of the selectivity of the present compounds. .

**Table 2. Comparative Activities in the Inhibition Assays**

| **No.** | **JNK1** | **JNK2** | **JNK3** | **p38** | **p38/JNK3** |
|---|---|---|---|---|---|
| II-65 | A | A | A | C | >10 |
| II-67 | A | A | A | C | >10 |
| II-70 | B | B | A | C | ≥10 |
| II-71 | A | A | A | C | >10 |
| II-72 | A | A | A | C | >10 |
| II-100 | A | A | A | C | >10 |
| II-101 | A | A | A | C | >10 |
| II-102 | A | A | A | C | >10 |

As can be seen from Table 2, the compounds of this invention have good activity against the three isoforms of JNK (JNK1, JNK2 and JNK3). The present compounds are much less active against p38 and provide good selectivity for JNK, as can be seen from the p38/JNK3 ratio. For typical compounds the inhibitory activity against JNK is estimated to be up to two or three orders of magnitude greater than the inhibitory activity against p38. By contrast, compounds that have larger G substituents (for example, where G is phenyl or 4-fluoro-phenyl) will be much more active against p38 and have poorer selectivity for the JNK enzyme.

While we have described a number of embodiments of this invention, it is apparent that our basic examples may be altered to provide other embodiments which utilize the compounds and methods of this invention. Therefore, it will be appreciated that the scope of this invention is to be defined by the appended claims rather than by the specific embodiments which have been represented by way of example.

## Claims

1. A compound of formula **II:** 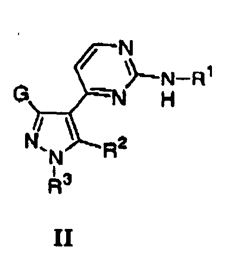 or a pharmaceutically acceptable salt thereof, wherein:
R¹ is selected from optionally substituted phenyl, cyclohexyl, pyridyl, naphthyl, or quinolinyl;
R is a C₁ to C₁₂ aliphatic or substituted aliphatic group;
R² is selected from hydrogen, or -R;
R³ is selected from R, hydroxyalkyl, alkoxyalkyl, alkylthioalkyl, aminoalkyl, alkylaminoalkyl, dialkylaminoalkyl, heterocyclyl, heterocyclylalkyl, aryl, aralkyl, or aryloxyalkyl;
G is hydrogen or C₁₋₃ alkyl;
wherein each substitutable carbon atom of said optionally substituted phenyl, cyclohexyl, pyridyl, naphthyl, or quinolinyl, including the fused ring when present, is optionally and independently substituted by halo, R, OR, SR, OH, NO₂, CN, NH₂, NHR, N(R)₂, NHCOR, NHCONHR, NHCON(R)₂, NRCOR, NHCO₂R, CO₂R, CO₂H, COR, CONHR, CON(R)₂, S(O)₂R, SONH₂, S(O)R, SO₂NHR, or NHS(O)₂R; and
wherein each substitutable nitrogen atom of said optionally substituted pyridyl or quinolinyl is optionally substituted by R, COR, S(O)₂R, or CO₂R.

2. A compound of claim 1 wherein G is hydrogen or methyl; R¹ is selected from phenyl, cyclohexyl, pyridyl, naphthyl, or quinolinyl; R² is selected from hydrogen, methyl, alkoxymethyl, benzyloxymethyl, or heterocyclylmethyl; and R³ is phenyl or benzyl; wherein each R¹-R³ is optionally substituted.

3. A compound of claim 2 wherein G is hydrogen or methyl; R¹ is phenyl or cyclohexyl; R² is methoxymethyl, methoxyethoxymethyl, ethoxymethyl, piperidin-1-ylmethyl, morpholin-4-ylmethyl, or tetrahydrofuran-3-ylmethyl; and R³ is phenyl or benzyl; wherein each R¹-R³ is optionally substituted.

4. A compound of claim 1, the compound having formula **II:** 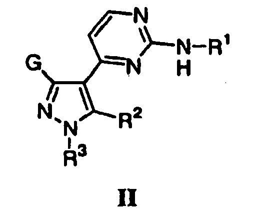 and selected from one of the compounds:
| **No.** | **G** | **R¹** | **R²** | **R³** |
|---|---|---|---|---|
| II-1 | CH₃ | Phenyl | H | Ph |
| II-2 | CH₃ | 4-methoxy-phenyl | H | Ph |
| II-3 | CH₃ | 3,4-dimethoxy-phenyl | H | Ph |
| II-4 | CH₃ | 3,5-dimethoxy-phenyl | H | Ph |
| II-5 | CH₃ | 4-cyano-phenyl | H | Ph |
| II-6 | CH₃ | 3-fluoro-phenyl | H | Ph |
| II-7 | CH₃ | 4-fluoro-phenyl | H | Ph |
| II-8 | CH₃ | 4-COCH₃-phenyl | H | Ph |
| II-9 | CH₃ | 4-CONH₂-phenyl | H | Ph |
| II-10 | CH₃ | 4-SCH₃-phenyl | H | Ph |
| II-11 | CH₃ | 3-OCH₃-phenyl | H | Ph |
| II-12 | CH₃ | 3,4,5-trimethoxy-phenyl | H | Ph |
| II-13 | CH₃ | 4-CO₂CH₃-phenyl | H | Ph |
| II-14 | CH₃ | 4-SO₂CH₃-phenyl | H | Ph |
| II-15 | CH₃ | 4-CO₂CH₃-phenyl | H | Ph |
| II-16 | CH₃ | 4-N(CH₃)₂-phenyl | H | Ph |
| II-17 | CH₃ | 3-NO₂-phenyl | H | Ph |
| II-18 | CH₃ | 3-NHCOCH₃-phenyl | H | Ph |
| II-19 | CH₃ | 3-NH₂-phenyl | H | Ph |
| II-20 | CH₃ | 4-NO₂-phenyl | H | Ph |
| II-21 | CH₃ | 3-(CH₂CH₂CO₂H)-phenyl | H | Ph |
| II-22 | CH₃ | 3-(CH₂CO₂H)-phenyl | H | Ph |
| II-23 | CH₃ | 3-CH₂OH-phenyl | H | 4-CH₃-Ph |
| II-24 | CH₃ | Phenyl | H | 4-OMe-phenyl |
| II-25 | CH₃ | 4-methoxy-phenyl | H | 4-OMe-phenyl |
| II-26 | CH₃ | 3,4-dimethoxy-phenyl | H | 4-Cl-Ph |
| II-27 | CH₃ | 3,5-dimethoxy-phenyl | H | 3,4-Cl₂-Ph |
| II-28 | CH₃ | 4-cyano-phenyl | H | 4-F-Ph |
| II-29 | CH₃ | 3-fluoro-phenyl | H | 4-OMe-phenyl |
| II-30 | CH₃ | 4-fluoro-phenyl | H | 2,5-Cl₂-PhPh |
| II-31 | CH₃ | 4-COCH₃-phenyl | H | 2,4-F₂-Ph |
| II-32 | CH₃ | 4-CONH₂-phenyl | H | 4-NO₂-Ph |
| II-33 | CH₃ | 4-SCH₃-phenyl | H | 3,5-Cl₂-Ph |
| II-34 | CH₃ | 3-OCH₃-phenyl | H | 3-Cl-Ph |
| II-35 | CH₃ | 3,4,5-trimethoxy-phenyl | H | 4-OMe-phenyl |
| II-36 | CH₃ | 4-CH₃-phenyl | H | 3-OBn-Ph |
| II-37 | CH₃ | cyclohexyl | H | 4-OMe-phenyl |
| II-38 | CH₃ | cyclohexyl | H | 4-OMe-phenyl |
| II-39 | CH₃ | cyclohexyl | H | 4-Cl-Ph |
| II-40 | CH₃ | cyclohexyl | H | 3,4-Cl₂-Ph |
| II-41 | CH₃ | cyclohexyl | H | 4-F-Ph |
| II-42 | CH₃ | cyclohexyl | H | 4-OMe-phenyl |
| II-43 | CH₃ | cyclohexyl | H | 2,5-Cl₂-Ph |
| II-44 | CH₃ | cyclohexyl | H | 2,4-F₂-Ph |
| II-45 | CH₃ | cyclohexyl | H | 4-NO₂-Ph |
| II-46 | CH₃ | cyclohexyl | H | 3,5-Cl₂-Ph |
| II-47 | CH₃ | cyclohexyl | H | 3-Cl-Ph |
| II-48 | CH₃ | cyclohexyl | H | 4-OMe-phenyl |
| II-49 | CH₃ | cyclohexyl | H | 3-OBn-Ph |
| II-50 | CH₃ | cyclohexyl | H | -CH₂Ph |
| II-51 | CH₃ | cyclohexyl | H | Ph |
| II-52 | CH₃ | Phenyl | H | 4-OMe-phenyl |
| II-53 | H | 4-methoxy-phenyl | H | 4-OMe-phenyl |
| II-54 | H | 3,4-dimethoxy-phenyl | H | 4-Cl-Ph |
| II-55 | H | 3,5-dimethoxy-phenyl | H | 3,4-Cl₂-Ph |
| II-56 | H | 4-cyano-phenyl | H | 4-F-Ph |
| II-57 | H | 3-fluoro-phenyl | H | 4-OMe-phenyl |
| II-58 | H | 4-fluoro-phenyl | H | 2,5-Cl₂-PhPh |
| II-59 | H | 4-COCH₃-phenyl | H | 2,4-F₂-Ph |
| II-60 | H | 4-CONH₂-phenyl | H | 4-NO₂-Ph |
| II-61 | H | 4-SCH₃-phenyl | H | 3,5-Cl₂-Ph |
| II-62 | H | 3-OCH₃-phenyl | H | ₃-Cl-Ph |
| II-63 | H | 3,4,5-trimethoxy-phenyl | H | 4-OMe-phenyl |
| II-64 | H | 4-CH₃-phenyl | H | 3-OBn-Ph |
| II-65 | H | cyclohexyl | H | benzyl |
| II-66 | H | cyclohexyl | H | 4-OMe-phenyl |
| II-67 | H | cyclohexyl | H | phenyl |
| II-68 | H | cyclohexyl | H | 3,4-Cl₂-Ph |
| II-69 | H | cyclohexyl | H | 2,4-Cl₂-Ph |
| II-70 | H | cyclohexyl | H | 4-OMe-phenyl |
| II-71 | H | cyclohexyl | H | 2,5-Cl₂-Ph |
| II-72 | H | cyclohexyl | H | 2,4-F₂-Ph |
| II-73 | H | cyclohexyl | H | 4-NO₂-Ph |
| II-74 | H | cyclohexyl | H | 3,5-Cl₂-Ph |
| II-75 | H | cyclohexyl | H | 3-Cl-Ph |
| II-76 | H | phenyl | H | 4-OMe-phenyl |
| II-78 | CH₃ | 4-methoxy-phenyl | H | -CH₂Ph |
| II-79 | CH₃ | 3,4-dimethoxy-phenyl | H | -CH₂Ph |
| II-80 | CH₃ | 3,5-dimethoxy-phenyl | H | -CH₂Ph |
| II-81 | CH₃ | 4-cyano-phenyl | H | -CH₂Ph |
| II-82 | CH₃ | 3-fluoro-phenyl | H | -CH₂Ph |
| II-83 | CH₃ | 3,4,5-trimethoxy-phenyl | H | -CH₂Ph |
| II-84 | CH₃ | 3-pyridyl | H | Ph |
| II-85 | CH₃ | 4-methoxy-pyrid-3-yl | H | Ph |
| II-86 | CH₃ | 2-naphthyl | H | Ph |
| II-88 | CH₃ | 6-methoxy-naphthalen-2-yl | H | Ph |
| II-90 | CH₃ | 2-methyl-quinolin-6-yl | H | Ph |
| II-91 | CH₃ | 4-methoxy-phenyl | CH₃ | Ph |
| II-92 | CH₃ | 3,4-dimethoxy-phenyl | CH₃ | Ph |
| II-93 | CH₃ | 3,5-dimethoxy-phenyl | CH₃ | 4-OMe-phenyl |
| II-94 | CH₃ | cyclohexyl | CH₃ | 4-OMe-phenyl |
| II-95 | CH₃ | cyclohexyl | CH₃ | 4-Cl-phenyl |
| II-96 | CH₃ | cyclohexyl | CH₃ | Ph |
| II-97 | CH₃ | 4-methoxy-phenyl | CH₃ | -CH₂Ph |
| II-98 | CH₃ | 2-methyl-quinolin-6-yl | CH₃ | -CH₂Ph |
| II-99 | CH₃ | 2-methyl-quinolin-6-yl | CH₃ | -CH₂Ph |
| II-100 | H | 4-F-phenyl | CH₃ | Ph |
| II-101 | H | 4-Cl-phenyl | CH₃ | Ph |
| II-102 | H | 4-NO₂-phenyl | CH₃ | Ph |
| II-103 | H | cyclohexyl | CH₃ | 2,6-difluoro-phenyl |
| II-104 | H | cyclohexyl | CH₃ | 3,5-dichloro-phenyl |
| II-105 | H | cyclohexyl | CH₃ | 2,4-dichloro-phenyl |
| II-106 | H | cyclohexyl | CH₃ | Ph |
| II-107 | H | 3-Cl-phenyl | CH₃ | Ph |
| II-108 | H | 3-benzyloxy-phenyl | CH₃ | Ph |
| II-109 | H | phenyl | CH₃ | 2,4-difluoro-phenyl |
| II-110 | CH₃ | 3-Cl-phenyl | H | phenyl |
| II-111 | H | phenyl | H | 2,4-difluoro-phenyl |
| II-112 | H | cyclohexyl | H | phenyl |
| II-113 | H | 3-Br-phenyl | CH₃ | _ phenyl |
| II-114 | H | 3-I-phenyl | CH₃ | phenyl |
| II-115 | H | 2-chloropyridin-5-yl | CH₃ | phenyl |
| II-116 | H | phenyl | CH₃ | pyridin-2-yl |
| II-117 | H | 4-F-phenyl | CH₃ | pyridin-2-yl |
| II-118 | H | 4-Cl-phenyl | CH₃ | pyridin-2-yl |
| II-119 | H | 3-Cl-phenyl | CH₃ | pyridin-2-yl |
| II-120 | H | 4-NO₂-phenyl | CH₃ | pyridin-2-yl |
| II-121 | H | 3-(benzyloxy)-phenyl | CH₃ | pyridin-2-yl |
| II-122 | H | 2,6-difluorophenyl | CH₃ | phenyl |
| II-123 | H | phenyl | CH₃ | 3-Cl-phenyl |
| II-124 | H | 4-F-phenyl | CH₃ | 3-Cl-phenyl |
| II-125 | H | 4-Cl-phenyl | CH₃ | 3-Cl-phenyl |
| II-126 | H | 3-Cl-phenyl | CH₃ | 3-Cl-phenyl |
| II-127 | H | 4-NO₂-phenyl | CH₃ | 3-Cl-phenyl |
| II-128 | H | 3-(benzyloxy)-phenyl | CH₃ | 3-Cl-phenyl |
| II-129 | H | naphthalen-2-yl | CH₃ | phenyl |
| II-130 | H | 3,4-dimethoxyphenyl | CH₃ | phenyl |
| II-131 | H | phenyl | CH₃ | 6-CH₃-4-CF₃-pyridin2-yl |
| II-132 | H | 4-F-phenyl | CH₃ | 6-CH₃-4-CF₃-pyridin2-yl |
| II-133 | H | 4-Cl-phenyl | CH₃ | 6-CH₃-4-CF₃-pyridin2-yl |
| II-134 | H | 3-Cl-phenyl | CH₃ | 6-CH₃-4-CF₃-pyridin2-yl |
| II-135 | H | 4-NO₂-phenyl | CH₃ | 6-CH₃-4-CF₃-pyridin2-yl |
| II-136 | H | 3-(benzyloxy)-phenyl | CH₃ | 6-CH₃-4-CF₃-pyridin2-yl |
| II-137 | H | 3-F-phenyl | CH₃ | pyridin-2-yl |
| II-138 | H | 3-chloro-4-methoxyphenyl | CH₃ | pyridin-2-yl |
| II-139 | H | naphthalen-2-yl | CH₃ | pyridin-2-yl |

5. A compound according to any one of claims 1 to 4 for use in treating a disease selected from inflammatory diseases, autoimmune diseases, destructive bone disorders, proliferative disorders, infectious diseases, neurodegenerative diseases, allergies, reperfusion/ischemia in stroke, heart attacks, angiogenic disorders, organ hypoxia, vascular hyperplasia, cardiac hypertrophy, thrombin-induced platelet aggregation, or conditions associated with proinflammatory cytokines.

6. A compound, for use according to claim 5, wherein said disease is an inflammatory disease selected from acute pancreatitis, chronic pancreatitis, asthma, allergies, or adult respiratory distress syndrome.

7. A compound, for use according to claim 5, wherein said disease is an autoimmune disease selected from glomerulonephritis, rheumatoid arthritis, systemic lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, or graft vs. host disease.

8. A compound, for use according to claim 5, wherein said disease is a destructive bone disorder selected from osteoarthritis, osteoporosis or multiple myeloma-related bone disorder.

9. A compound, for use according to claim 5, wherein said disease is a proliferative disease selected from acute myelogenous leukemia, chronic myelogenous leukemia, metastatic melanoma, Kaposi's sarcoma, or multiple myeloma.

10. A compound, for use according to claim 5, wherein said disease is a neurodegenerative disease selected from Alzheimer's disease, Parkinson's disease, amyotrophic lateral sclerosis, Huntington's disease, cerebral ischemia or neurodegenerative disease caused by traumatic injury, glutamate neurotoxicity or hypoxia.

11. A compound, for use according to claim 5, wherein said disease is selected from ischemia/reperfusion in stroke or myocardial ischemia, renal ischemia, heart attacks, organ hypoxia or thrombin-induced platelet aggregation.

12. A compound, for use according to claim 5, wherein said disease is a condition associated with T-cell activation or pathologic immune responses.

13. A compound, for use according to claim 5, wherein said disease is an angiogenic disorder selected from solid tumors, ocular neovasculization, or infantile haemangioma.

14. A composition comprising a compound according to any one of claims 1 to 4 or a pharmaceutically acceptable salt thereof, and a pharmaceutically acceptable carrier, vehicle, or diluent.

## Patentansprüche

1. Eine Verbindung der Formel II: 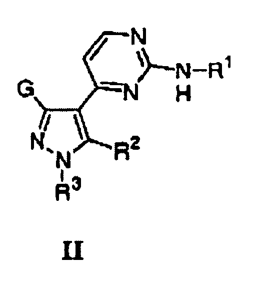 oder ein pharmazeutisch verträgliches Salz davon, wobei:
R¹ ausgewählt ist aus gegebenenfalls substituiertem Phenyl,
Cyclohexyl, Pyridyl, Naphtyl oder Quinolinyl;
R eine C₁ bis C₁₂ aliphatische oder substituierte aliphatische Gruppe ist;
R² ausgewählt ist aus Wasserstoff oder -R;
R³ ausgewählt ist aus R, Hydroxyalkyl, Alkoxyalkyl, Alkylthioalkyl, Aminoalkyl, Alkylaminoalkyl, Dialkylaminoalkyl, Heterocyclyl, Heterocyclylalkyl, Aryl, Aralkyl oder Aryloxyalkyl; G Wasserstoff oder C₁₋₃ alkyl ist;
wobei jedes substituierbare Kohlenstoffatom des gegebenenfalls substituierten Phenyl, Cyclohexyl, Pyridyl, Naphtyl, oder Quinolinyl, einschließlich des fusionierten Rings, falls vorhanden, gegebenenfalls und unabhängig substituiert ist durch Halo, R, OR, SR, OH, NO₂, CN, NH₂, NHR, N(R)₂, NHCOR, NHCONHR, NHCON(R)₂, NRCOR, NHCO₂R, CO₂R, CO₂H, COR, CONHR, CON(R)₂, S(O)₂R, SONH₂, S(O)R, SO₂NHR, oder NHS(O)₂R; und wobei jedes substituierbare Stickstoffatom des gegebenenfalls substituierten Pyridyl oder Quinolinyl gegebenenfalls substituiert ist durch R, COR, S(O)₂R oder CO₂R.

2. Eine Verbindung nach Anspruch 1, wobei G Wasserstoff oder Methyl ist; R¹ ausgewählt ist aus Phenyl, Cyclohexyl, Pyridyl, Naphtyl oder Quinolinyl; R² ausgewählt ist aus Wasserstoff, Methyl, Alkoxymethyl, Benzyloxymethyl oder Heterocyclylmethyl; und R³ Phenyl oder Benzyl ist, wobei jeder R¹-R³ gegebenenfalls substituiert ist.

3. Eine Verbindung nach Anspruch 2, wobei G Wasserstoff oder Methyl ist; R¹ Phenyl oder Cyclohexyl ist; R² Methoxymethyl, Methoxyethoxymethyl, Ethoxymethyl, Piperididn-1-ylmethyl, Morpholin-4-ylmethyl oder Tetrahydrofuran-3-ylmethyl ist; und R³ Phenyl oder Benzyl ist; wobei jeder der R¹-R³ gegebenenfalls substituiert ist.

4. Eine Verbindung nach Anspruch 1, wobei die Verbindung die Formel II hat: 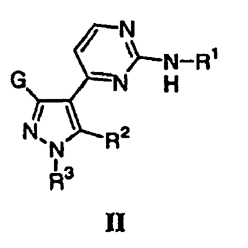 und ausgewählt ist aus einer der Verbindungen:
| **Nr.** | **G** | **R¹** | **R²** | **R³** |
|---|---|---|---|---|
| II-1 | CH₃ | Phenyl | H | Ph |
| II-2 | CH₃ | 4-methoxy-phenyl | H | Ph |
| II-3 | CH₃ | 3,4-dimethoxy-phenyl | H | Ph |
| II-4 | CH₃ | 3,5-dimethoxy-phenyl | H | Ph |
| II-5 | CH₃ | 4-cyano-phenyl | H | Ph |
| **Nr.** | **G** | **R¹** | **R²** | **R³** |
| II-6 | CH₃ | 3-fluor-phenyl | H | Ph |
| II-7 | CH₃ | 4-fluor-phenyl | H | Ph |
| II-8 | CH₃ | 4-COCH₃-phenyl | H | Ph |
| II-9 | CH₃ | 4-CONH₂-phenyl | H | Ph |
| II-10 | CH₃ | 4-SCH₃-phenyl | H | Ph |
| II-11 | CH₃ | 3-OCH₃-phenyl | H | Ph |
| II-12 | CH₃ | 3,4,5-trimethoxy-phenyl | H | Ph |
| II-13 | CH₃ | 4-CO₂CH₃-phenyl | H | Ph |
| II-14 | CH₃ | 4-SO₂CH₃-phenyl | H | Ph |
| II-15 | CH₃ | 4-CO₂CH₃-phenyl | H | Ph |
| II-16 | CH₃ | 4-N(CH₃)₂-phenyl | H | Ph |
| II-17 | CH₃ | 3-NO₂-phenyl | H | Ph |
| II-18 | CH₃ | 3-NHCOCH₃-phenyl | H | Ph |
| II-19 | CH₃ | 3-NH₂-phenyl | H | Ph |
| II-20 | CH₃ | 4-NO₂-phenyl | H | Ph |
| II-21 | CH₃ | 3- (CH₂CH₂CO₂H) -phenyl | H | Ph |
| II-22 | CH₃ | 3-(CH₂CO₂H)-phenyl | H | Ph |
| II-23 | CH₃ | 3-CH₂OH-phenyl | H | 4-CH₃-Ph |
| II-24 | CH₃ | Phenyl | H | 4-OMe-phenyl |
| II-25 | CH₃ | 4-methoxy-phenyl | H | 4-OMe-phenyl |
| II-26 | CH₃ | 3,4-dimethoxy-phenyl | H | 4-Cl-Ph |
| II-27 | CH₃ | 3,5-dimethoxy-phenyl | H | 3,4-Cl₂-Ph |
| II-28 | CH₃ | 4-cyano-phenyl | H | 4-F-Ph |
| II-29 | CH₃ | 3-flour-phenyl | H | 4-OMe-phenyl |
| II-30 | CH₃ | 4-flour-phenyl | H | 2,5-Cl₂PhPh |
| II-31 | CH₃ | 4-COCH₃-phenyl | H | 2,4-F₂-Ph |
| II-32 | CH₃ | 4-CONH₂-phenyl | H | 4-NO₂-Ph |
| II-33 | CH₃ | 4-SCH₃-phenyl | H | 3,5-Cl₂-Ph |
| II-34 | CH₃ | 3-OCH₃-phenyl | H | 3-Cl-Ph |
| II-35 | CH₃ | 3,4,5-trimethoxy-phenyl | H | 4-OMe-phenyl |
| II-36 | CH₃ | 4-CH₃-phenyl | H | 3-Obn-Ph |
| II-37 | CH₃ | Cyclohexyl | H | 4-OMe-phenyl |
| II-38 | CH₃ | Cyclohexyl | H | 4-OMe-phenyl |
| II-39 | CH₃ | Cyclohexyl | H | 4-Cl-Ph |
| II-40 | CH₃ | Cyclohexyl | H | 3,4-Cl₂-Ph |
| II-41 | CH₃ | Cyclohexyl | H | 4-F-Ph |
| II-42 | CH₃ | Cyclohexyl | H | 4-OMe-phenyl |
| **Nr.** | **G** | **R¹** | **R²** | **R³** |
| II-43 | CH₃ | Cyclohexyl | H | 2,5-Cl₂-Ph |
| II-44 | CH₃ | Cyclohexyl | H | 2,4-F₂-Ph |
| II-45 | CH₃ | Cyclohexyl | H | 4-NO₂-Ph |
| II-46 | CH₃ | Cyclohexyl | H | 3,5-Cl₂-Ph |
| II-47 | CH₃ | Cyclohexyl | H | 3-Cl-Ph |
| II-48 | CH₃ | Cyclohexyl | H | 4-OMe-phenyl |
| II-49 | CH₃ | Cyclohexyl | H | 3-Obn-Ph |
| II-50 | CH₃ | Cyclohexyl | H | -CH₂Ph |
| II-51 | CH₃ | Cyclohexyl | H | Ph |
| II-52 | CH₃ | Phenyl | H | 4-OMe-phenyl |
| II-53 | H | 4-methoxy-phenyl | H | 4-OMe-phenyl |
| II-54 | H | 3,4-dimethoxy-phenyl | H | 4-Cl-Ph |
| II-55 | H | 3,5-dimethoxy-phenyl | H | 3,4-Cl₂-Ph |
| II-56 | H | 4-cyano-phenyl | H | 4-F-Ph |
| II-57 | H | 3-fluor-phenyl | H | 4-OMe-phenyl |
| II-58 | H | 4-fluor-phenyl | H | 2,5-Cl₂-PhPh |
| II-59 | H | 4-COCH₃-phenyl | H | 2,4-F₂-Ph |
| II-60 | H | 4-CONH₂-phenyl | H | 4-NO₂-Ph |
| II-61 | H | 4-SCH₃-phenyl | H | 3,5-Cl₂-Ph |
| II-62 | H | 3-OCH₃-phenyl | H | 3-Cl-Ph |
| II-63 | H | 3,4,5-trimethoxy-phenyl | H | 4-OMe-phenyl |
| II-64 | H | 4-CH₃-phenyl | H | 3-Obn-Ph |
| II-65 | H | Cyclohexyl | H | Benzyl |
| II-66 | H | Cyclohexyl | H | 4-OMe-phenyl |
| II-67 | H | Cyclohexyl | H | Phenyl |
| II-68 | H | Cyclohexyl | H | 3,4-Cl₂-Ph |
| II-69 | H | Cyclohexyl | H | 2,4-Cl₂-Ph |
| II-70 | H | Cyclohexyl | H | 4-OMe-phenyl |
| II-71 | H | Cyclohexyl | H | 2,5-Cl₂-Ph |
| II-72 | H | Cyclohexyl | H | 2,4-F₂-Ph |
| II-73 | H | Cyclohexyl | H | 4-NO₂₋Ph |
| II-74 | H | Cyclohexyl | H | 3,5-Cl₂-Ph |
| II-75 | H | Cyclohexyl | H | 3-Cl-Ph |
| II-76 | H | Phenyl | H | 4-OMe-phenyl |
| II-78 | CH₃ | 4-methoxy-phenyl | H | -CH₂Ph |
| II-79 | CH₃ | 3,4-dimethoxy-phenyl | H | -CH₂Ph |
| II-80 | CH₃ | 3,5-dimethoxy-phenyl | H | -CH₂Ph |
| **Nr.** | **G** | **R¹** | **R²** | **R³** |
| II-81 | CH₃ | 4-cyano-phenyl | H | -CH₂Ph |
| II-82 | CH₃ | 3-fluor-phenyl | H | -CH₂Ph |
| II-83 | CH₃ | 3,4,5-trimethoxy-phenyl | H | -CH₂Ph |
| II-84 | CH₃ | 3-pyridyl | H | Ph |
| II-85 | CH₃ | 4-methoxy-pyrid-3-yl | H | Ph |
| II-86 | CH₃ | 2-naphthyl | H | Ph |
| II-88 | CH₃ | 6-methoxy-naphthalen-2-yl | H | Ph |
| II-90 | CH₃ | 2-methyl-quinolin-6-yl | H | Ph |
| II-91 | CH₃ | 4-methoxy-phenyl | CH₃ | Ph |
| II-92 | CH₃ | 3,4-dimethoxy-phenyl | CH₃ | Ph |
| II-93 | CH₃ | 3,5-dimethoxy-phenyl | CH₃ | 4-OMe-phenyl |
| II-94 | CH₃ | Cyclohexyl | CH₃ | 4-OMe-phenyl |
| II-95 | CH₃ | Cyclohexyl | CH₃ | 4-Cl-phenyl |
| II-96 | CH₃ | Cyclohexyl | CH₃ | Ph |
| II-97 | CH₃ | 4-methoxy-phenyl | CH₃ | -CH₂Ph |
| II-98 | CH₃ | 2-methyl-quinolin-6-yl | CH₃ | -CH₂Ph |
| II-99 | CH₃ | 2-methyl-quinolin-6-yl | CH₃ | -CH₂Ph |
| II-100 | H | 4-F-phenyl | CH₃ | Ph |
| II-101 | H | 4-Cl-phenyl | CH₃ | Ph |
| II-102 | H | 4 -NO₂-phenyl | CH₃ | Ph |
| II-103 | H | Cyclohexyl | CH₃ | 2,6-difluor-phenyl |
| II-104 | H | Cyclohexyl | CH₃ | 3,5-dichlor-phenyl |
| II-105 | H | Cyclohexyl | CH₃ | 2,4-dichlor-phenyl |
| II-106 | H | Cyclohexyl | CH₃ | Ph |
| II-107 | H | 3-Cl-phenyl | CH₃ | Ph |
| II-108 | H | 3-benzyloxy-phenyl | CH₃ | Ph |
| II-109 | H | Phenyl | CH₃ | 2,4-difluor-phenyl |
| II-110 | CH₃ | 3-Cl-phenyl | H | Phenyl |
| II-111 | H | Phenyl | H | 2,4-difluor-phenyl |
| II-112 | H | Cyclohexyl | H | Phenyl |
| II-113 | H | 3-Br-phenyl | CH₃ | Phenyl |
| II-114 | H | 3-I-phenyl | CH₃ | Phenyl |
| II-115 | H | 2-chlorpyridin-5-yl | CH₃ | Phenyl |
| II-116 | H | Phenyl | CH₃ | Pyridin-2-yl |
| II-117 | H | 4-F-phenyl | CH₃ | Pyridin-2-yl |
| II-118 | H | 4-Cl-phenyl | CH₃ | Pyridin-2-yl |
| II-119 | H | 3-Cl-phenyl | CH₃ | Pyridin-2-yl |
| **Nr.** | **G** | **R¹** | **R²** | **R³** |
| II-120 | H | 4-NO₂-phenyl | CH₃ | Pyridin-2-yl |
| II-121 | H | 3-(benzyloxy)-phenyl | CH₃ | Pyridin-2-yl |
| II-122 | H | 2,6-difluorphenyl | CH₃ | Phenyl |
| II-123 | H | Phenyl | CH₃ | 3-Cl-phenyl |
| II-124 | H | 4-F-phenyl | CH₃ | 3-Cl-phenyl |
| II-125 | H | 4-Cl-phenyl | CH₃ | 3-Cl-phenyl |
| II-126 | H | 3-Cl-phenyl | CH₃ | 3-Cl-phenyl |
| II-127 | H | 4-NO₂-phenyl | CH₃ | 3-Cl-phenyl |
| II-128 | H | 3-(benzyloxy)-phenyl | CH₃ | 3-Cl-phenyl |
| II-129 | H | Naphthalen-2-yl | CH₃ | Phenyl |
| II-130 | H | 3,4-dimethoxy-phenyl | CH₃ | Phenyl |
| II-131 | H | Phenyl | CH₃ | 6-CH₃-4-CF₃-pyridin2-yl |
| II-132 | H | 4-F-phenyl | CH₃ | 6-CH₃-4-CF₃-pyridin2-yl |
| II-133 | H | 4-Cl-phenyl | CH₃ | 6-CH₃-4-CF₃-pyridin2-yl |
| II-134 | H | 3-Cl-phenyl | CH₃ | 6-CH₃-4-CF₃-pyridin2-yl |
| II-135 | H | 4-NO₂-phenyl | CH₃ | 6-CH₃-4-CF₃-pyridin2-yl |
| II-136 | H | 3-(benzyloxy)-phenyl | CH₃ | 6-CH₃-4-CF₃-pyridin2-yl |
| II-137 | H | 3-F-phenyl | CH₃ | Pyridin-2-yl |
| II-138 | H | 3-chlor-4-methoxyphenyl | CH₃ | Pyridin-2-yl |
| II-139 | H | Naphthalen-2-yl | CH₃ | Pyridin-2-yl |

5. Eine Verbindung nach einem der Ansprüche 1 bis 4 zur Verwendung in der Behandlung einer Krankheit ausgewählt aus inflammatorischen Krankheiten, Autoimmunkrankheiten, destruktiven Knochenerkrankungen, proliferativen Erkrankungen, Infektionskrankheiten, neurodegenerativen Krankheiten, Allergien, Reperfusion/Ischämie in Schlaganfall, Herzattacken, angiogenen Erkrankungen, Organhypoxie, vaskuläre Hyperplasie, Herzhypertrophie, thrombininduzierte Blutplättchenaggregation, oder mit proinflammatorischen Cytokinen assoziierten Beschwerden.

6. Eine Verbindung zur Verwendung nach Anspruch 5, wobei diese Krankheit eine inflammatorische Krankheit ist ausgewählt aus akuter Pankreatitis, chronischer Pankreatitis, Asthma, Allergien, oder Schocklunge.

7. Eine Verbindung zur Verwendung nach Anspruch 5, wobei diese Krankheit eine Autoimmunkrankheit ist ausgewählt aus Glomerulonephritis, rheumatoider Arthritis, systemischem Lupus, Sclerodermie, chronischer Thyroiditis, Basedow'schen Krankheit, autoimmuner Gastritis, Diabetes, Autoagglutinationsanämie, autoimmuner Neutropenie, Thrombocytopenie, atopischer Dermatitis, chronisch aktiver Hepatitis, Myasthenia gravis, multipler Sklerose, entzündlicher Darmerkrankung, Colitis ulcerosa, Crohn'scher Krankheit, Psoriasis, oder Transplantat-Empfänger-Reaktion.

8. Eine Verbindung zur Verwendung nach Anspruch 5, wobei diese Krankheit eine destruktive Knochenerkrankung ist ausgewählt aus Osteoarthritis, Osteoporose, oder multiples Myelombezogener Knochenerkrankung.

9. Eine Verbindung zur Verwendung nach Anspruch 5, wobei diese Krankheit eine prolifertaive Krankheit ist ausgewählt aus akuter myelogener Leukämie, chronischer myelogener Leukämie, metastatischem Melanom, Kaposi's Sarkom, oder multiplem Myelom.

10. Eine Verbindung zur Verwendung nach Anspruch 5, wobei diese Krankheit eine neurodegenerative Krankheit ist ausgewählt aus Alzheimer'scher Krankheit, Parkinson-Krankheit, a-myotropher Lateralsklerose, Huntington'schen-Krankheit, cerebraler Ischämie oder neurodegenerativer Krankheit, verursacht durch traumatische Verletzungen, Glutamatneurotoxizität oder Hypoxie.

11. Eine Verbindung zur Verwendung nach Anspruch 5, wobei diese Krankheit ausgewählt ist aus Ischämie/Reperfusion in Schlaganfall oder myokardialer Ischämie, renaler Ischämie, Herzattacken, Organhypoxie, oder thrombininduzierter Blutplättchenaggregation.

12. Eine Verbindung zur Verwendung nach Anspruch 5, wobei diese Krankheit ein Befinden ist, assoziiert mit T-Zell-Aktivierung oder pathologischen Immunantworten.

13. Eine Verbindung zur Verwendung nach Anspruch 5, wobei diese Krankheit eine angiogene Erkrankung ist ausgewählt aus soliden Tumoren, okularer Neovaskulisierung, oder infantilem Hämangiom.

14. Eine Zusammensetzung, umfassend eine Verbindung nach einem der Ansprüche 1 bis 4 oder ein pharmazeutisch verträgliches Salz davon und ein/en pharmazeutisch verträglichen Träger, Vehikel oder Verdünnungsmittel.

## Revendications

1. Composé de formule II 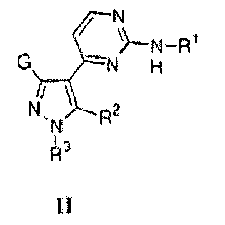 ou un de ses sels pharmaceutiquement acceptables, formule dans laquelle :
R¹ est choisi entre des groupes phényle, cyclohexyle, pyridyle, naphtyle et quinolinyle facultativement substitués ;
R représente un groupe en C₁ à C₁₂ aliphatique ou aliphatique substitué ;
R² est choisi entre un atome d'hydrogène et un groupe -R ;
R³ est choisi entre des groupes R, hydroxyalkyle, alkoxyalkyle, alkylthioalkyle, aminoalkyle, alkylaminoalkyle, dialkylaminoalkyle, hétérocyclyle, hétérocyclylalkyle, aryle, aralkyle ou aryloxyalkyle ;
G représente un atome d'hydrogène ou un groupe alkyle en C₁ à C₃ ;
dans laquelle chaque atome de carbone substituable dudit groupe phényle, cyclohexyle, pyridyle, naphtyle ou quinolinyle facultativement substitué, y compris le noyau condensé lorsqu'il est présent, est facultativement et indépendamment substitué avec un substituant halogéno, R, OR, SR, OH, NO₂, CN, NH₂, NHR, N(R)₂, NHCOR, NHCONHR, NHCON(R)₂, NRCOR, NHCO₂R, CO₂R, CO₂H, COR, CONHR, CON(R)₂, S(O)₂R, SONH₂, S(O)R, SO₂NHR ou NHS(O)₂R ; et
dans laquelle chaque d'atome d'azote substituable dudit groupe pyridyle ou quinolinyle facultativement substitué est facultativement substitué avec un substituant R, COR, S(O)₂R ou CO₂R.

2. Composé suivant la revendication 1, dans lequel G représente un atome d'hydrogène ou un groupe méthyle ; R¹ est choisi entre des groupes phényle, cyclohexyle, pyridyle, naphtyle et quinolinyle ; R² est choisi entre un atome d'hydrogène, des groupes méthyle, alkoxyméthyle, benzyloxyméthyle et hétérocyclylméthyle ; et R³ représente un groupe phényle ou benzyle ; chacun des groupes R¹ à R³ étant facultativement substitué.

3. Composé suivant la revendication 2, dans lequel G représente un atome d'hydrogène ou un groupe méthyle ; R¹ représente un groupe phényle ou cyclohexyle ; R² représente un groupe méthoxyméthyle, méthoxyéthoxyméthyle, éthoxyméthyle, pipéridine-1-ylméthyle, morpholine-4-ylméthyle ou tétrahydrofuranne-3-ylméthyle ; et R³ représente un groupe phényle ou benzyle ; chacun des groupes R¹ à R³ étant facultativement substitué.

4. Composé suivant la revendication 1, ledit composé répondant à la formule II : 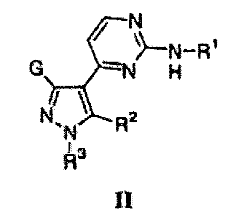 et étant un composé choisi parmi les suivants :
| **N°** | **G** | **R¹** | **R²** | **R³** |
|---|---|---|---|---|
| II-1 | CH₃ | phényle | H | Ph |
| II-2 | CH₃ | 4-méthoxy-phényle | H | Ph |
| II-3 | CH₃ | 3,4-diméthoxy-phényle | H | Ph |
| II-4 | CH₃ | 3,5-diméthoxy-phényle | H | Ph |
| II-5 | CH₃ | 4-cyano-phényle | H | Ph |
| II-6 | CH₃ | 3-fluoro-phényle | H | Ph |
| II-7 | CH₃ | 4-fluoro-phényle | H | Ph |
| II-8 | CH₃ | 4-COCH₃-phényle | H | Ph |
| II-9 | CH₃ | 4-CONH₂-phényle | H | Ph |
| II-10 | CH₃ | 4-SCH₃-phényle | H | Ph |
| II-11 | CH₃ | 3-OCH₃-phényle | H | Ph |
| II-12 | CH₃ | 3,4,5-triméthoxy-phényle | H | Ph |
| II-13 | CH₃ | 4-CO₂CH₃-phényle | H | Ph |
| II-14 | CH₃ | 4-SO₂CH₃-phényle | H | Ph |
| II-15 | CH₃ | 4-CO₂CH₃-phényle | H | Ph |
| II-16 | CH₃ | 4-N(CH₃)₂-phényle | H | Ph |
| II-17 | CH₃ | 3-NO₂-phényle | H | Ph |
| II-18 | CH₃ | 3-NHCOCH₃-phényle | H | Ph |
| II-19 | CH₃ | 3-NH₂-phényle | H | Ph |
| II-20 | CH₃ | 4-NO₂-phényle | H | Ph |
| II-21 | CH₃ | 3-(CH₂CH₂CO₂H)-phényle | H | Ph |
| II-22 | CH₃ | 3-(CH₂CO₂H)-phényle | H | Ph |
| II-23 | CH₃ | 3-CH₂OH-phényle | H | 4-CH₃-Ph |
| II-24 | CH₃ | phényle | H | 4-OMe-phényle |
| II-25 | CH₃ | 4-méthoxy-phényle | H | 4-OMe-phényle |
| II-26 | CH₃ | 3,4-diméthoxy-phényle | H | 4-Cl-Ph |
| II-27 | CH₃ | 3,5-diméthoxy-phényle | H | 3,4-Cl₂-Ph |
| II-28 | CH₃ | 4-cyano-phényle | H | 4-F-Ph |
| II-29 | CH₃ | 3-fluoro-phényle | H | 4-OMe-phényle |
| II-30 | CH₃ | 4-fluoro-phényle | H | 2,5-Cl₂-PhPh |
| II-31 | CH₃ | 4-COCH₃-phényle | H | 2,4-F₂-Ph |
| II-32 | CH₃ | 4-CONH₂-phényle | H | 4-NO₂-Ph |
| II-33 | CH₃ | 4-SCH₃-phényle | H | 3,5-Cl₂-Ph |
| II-34 | CH₃ | 3-OCH₃-phényle | H | 3-Cl-Ph |
| II-35 | CH₃ | 3,4,5-triméthoxy-phényle | H | 4-OMe-phényle |
| II-36 | CH₃ | 4-CH₃-phényle | H | 3-OBn-Ph |
| II-37 | CH₃ | cyclohexyle | H | 4-OMe-phényle |
| II-38 | CH₃ | cyclohexyle | H | 4-OMe-phényle |
| II-39 | CH₃ | cyclohexyle | H | 4-Cl-Ph |
| II-40 | CH₃ | cyclohexyle | H | 3,4-Cl₂-Ph |
| II-41 | CH₃ | cyclohexyle | H | 4-F-Ph |
| II-42 | CH₃ | cyclohexyle | H | 4-OMe-phényle |
| II-43 | CH₃ | cyclohexyle | H | 2,5-Cl₂-Ph |
| II-44 | CH₃ | cyclohexyle | H | 2,5-F₂-Ph |
| II-45 | CH₃ | cyclohexyle | H | 4-NO₂-Ph |
| II-46 | CH₃ | cyclohexyle | H | 3,5-Cl₂-Ph |
| II-47 | CH₃ | cyclohexyle | H | 3-Cl-Ph |
| II-48 | CH₃ | cyclohexyle | H | 4-OMe-phényle |
| II-49 | CH₃ | cyclohexyle | H | 3-OBn-Ph |
| II-50 | CH₃ | cyclohexyle | H | -CH₂Ph |
| II-51 | CH₃ | cyclohexyle | H | Ph |
| II-52 | CH₃ | phényle | H | 4-OMe-phényle |
| II-53 | H | 4-méthoxy-phényle | H | 4-OMe-phényle |
| II-54 | H | 3,4-diméthoxy-phényle | H | 4-Cl-Ph |
| II-55 | H | 3,5-diméthoxy-phényle | H | 3,4-Cl₂-Ph |
| II-56 | H | 4-cyano-phényle | H | 4-F-Ph |
| II-57 | H | 3-fluoro-phényle | H | 4-OMe-phényle |
| II-58 | H | 4-fluoro-phényle | H | 2,5-Cl₂-Ph-Ph |
| II-59 | H | 4-COCH₃-phényle | H | 2,4-F₂-Ph |
| II-60 | H | 4-CONH₂-phényle | H | 4-NO₂-Ph |
| II-61 | H | 4-SCH₃-phényle | H | 3,5-Cl₂-Ph |
| II-62 | H | 3-OCH₃-phényle | H | 3-Cl-Ph |
| II-63 | H | 3,4,5-triméthoxy-phényle | H | 4-OMe-phényle |
| II-64 | H | 4-CH₃-phényle | H | 3-OBn-Ph |
| II-65 | H | cyclohexyle | H | benzyle |
| II-66 | H | cyclohexyle | H | 4-OMe-phényle |
| II-67 | H | cyclohexyle | H | phényle |
| II-68 | H | cyclohexyle | H | 3,4-Cl₂-Ph |
| II-69 | H | cyclohexyle | H | 3,4-Cl₂-Ph |
| II-70 | H | cyclohexyle | H | 4-OMe-phényle |
| II-71 | H | cyclohexyle | H | 2,5-Cl₂-Ph |
| II-72 | H | cyclohexyle | H | 2,4-F₂-Ph |
| II-73 | H | cyclohexyle | H | 4-NO₂-Ph |
| II-74 | H | cyclohexyle | H | 3,5-Cl₂-Ph |
| II-75 | H | cyclohexyle | H | 3-Cl-Ph |
| II-76 | H | phényle | H | 4-OMe-phényle |
| II-78 | CH₃ | 4-méthoxy-phényle | H | -CH₂Ph |
| II-79 | CH₃ | 3,4-diméthoxy-phényle | H | -CH₂-Ph |
| II-80 | CH₃ | 3,5-diméthoxy-phényle | H | -CH₂-Ph |
| II-81 | CH₃ | 4-cyano-phényle | H | -CH₂Ph |
| II-82 | CH₃ | 3-fluoro-phényle | H | -CH₂Ph |
| II-83 | CH₃ | 3,4,5-triméthoxy-phényle | H | -CH₂Ph |
| II-84 | CH₃ | 3-pyridyle | H | Ph |
| II-85 | CH₃ | 4-méthoxy-pyrid-3-yle | H | Ph |
| II-86 | CH₃ | 2-naphtyle | H | Ph |
| II-88 | CH₃ | 6-méthoxy-naphtalène-2-yle | H | Ph |
| II-90 | CH₃ | 2-méthyl-quinoléine-6-yle | H | Ph |
| II-91 | CH₃ | 4-méthoxy-phényle | CH₃ | Ph |
| II-92 | CH₃ | 3,4-diméthoxy-phényle | CH₃ | Ph |
| II-93 | CH₃ | 3,5-diméthoxy-phényle | CH₃ | 4-OMe-phényle |
| II-94 | CH₃ | cyclohexyle | CH₃ | 4-OMe-phényle |
| II-95 | CH₃ | cyclohexyle | CH₃ | 4-Cl-phényle |
| II-96 | CH₃ | cyclohexyle | CH₃ | Ph |
| II-97 | CH₃ | 4-méthoxy-phényle | CH₃ | -CH₂Ph |
| II-98 | CH₃ | 2-méthyl-quinoléine-6-yle | CH₃ | -CH₂Ph |
| II-99 | CH₃ | 2-méthyl-quinoléine-6-yle | CH₃ | -CH₂Ph |
| II-100 | H | 4-F-phényle | CH₃ | Ph |
| II-101 | H | 4-Cl-phényle | CH₃ | Ph |
| II-102 | H | 4-NO₂-phényle | CH₃ | Ph |
| II-103 | H | cyclohexyle | CH₃ | 2,6-difluoro-phényle |
| II-104 | H | cyclohexyle | CH₃ | 3,5-dichloro-phényle |
| II-105 | H | cyclohexyle | CH₃ | 2,4-dichloro-phényle |
| II-106 | H | cyclohexyle | CH₃ | Ph |
| II-107 | H | 3-Cl-phényle | CH₃ | Ph |
| II-108 | H | 3-benzyloxy-phényle | CH₃ | Ph |
| II-109 | H | phényle | CH₃ | 2,4-difluoro-phényle |
| II-110 | CH₃ | 3-Cl-phényle | H | phényle |
| II-111 | H | phényle | H | 2,4-difluoro-phényle |
| II-112 | H | cyclohexyle | H | phényle |
| II-113 | H | 3-Br-phényle | CH₃ | phényle |
| II-114 | H | 3-I-phényle | CH₃ | phényle |
| II-115 | H | 2-chloropyridine-5-yle | CH₃ | phényle |
| II-116 | H | phényle | CH₃ | pyridine-2-yle |
| II-117 | H | 4-F-phényle | CH₃ | pyridine-2-yle |
| II-118 | H | 4-Cl-phényle | CH₃ | pyridine-2-yle |
| II-119 | H | 3-Cl-phényle | CH₃ | pyridine-2-yle |
| II-120 | H | 4-NO₂-phényle | CH₃ | pyridine-2-yle |
| II-121 | H | 3-(benzyloxy)-phényle | CH₃ | pyridine-2-yle |
| II-122 | H | 2,6-difluorophényle | CH₃ | phényle |
| II-123 | H | phényle | CH₃ | 3-Cl-phényle |
| II-124 | H | 4-F-phényle | CH₃ | 3-Cl-phényle |
| II-125 | H | 4-Cl-phényle | CH₃ | 3-Cl-phényle |
| II-126 | H | 3-Cl-phényle | CH₃ | 3-Cl-phényle |
| II-127 | H | 4-NO₂-phényle | CH₃ | 3-Cl-phényle |
| II-128 | H | 3-(benzyloxy)-phényle | CH₃ | 3-Cl-phényle |
| II-129 | H | naphtalène-2-yle | CH₃ | phényle |
| II-130 | H | 3,4-diméthoxyphényle | CH₃ | phényle |
| II-131 | H | phényle | CH₃ | 6-CH₃-4-CF₃-pyridine-2-yle |
| II-132 | H | 4-F-phényle | CH₃ | 6-CH₃-4-CF₃-pyridine-2-yle |
| II-133 | H | 4-Cl-phényle | CH₃ | 6-CH₃-4-CF₃-pyridine-2-yle |
| II-134 | H | 3-Cl-phényle | CH₃ | 6-CH₃-4-CF₃-pyridine-2-yle |
| II-135 | H | 4-NO₂-phényle | CH₃ | 6-CH₃-4-CF₃-pyridine-2-yle |
| II-136 | H | 3-(benzyloxy)-phényle | CH₃ | 6-CH₃-4-CF₃-pyridine-2-yle |
| II-137 | H | 3-F-phényle | CH₃ | pyridine-2-yle |
| II-138 | H | 3-chloro-4-méthoxyphényle | CH₃ | pyridine-2-yle |
| II-139 | H | naphtalène-2-yle | CH₃ | pyridine-2-yle |

5. Composé suivant l'une quelconque des revendications 1 à 4, destiné à être utilisé dans le traitement d'une maladie choisie entre des maladies inflammatoires, des maladies auto-immunes, des troubles osseux destructeurs, des troubles prolifératifs, des maladies infectieuses, des maladies neurodégénératives, des allergies, une reperfusion/ischémie dans un ictus, des attaques cardiaques, des troubles angiogéniques, une hypoxie d'organe, l'hyperplasie vasculaire, l'hypertrophie cardiaque, l'agrégation plaquettaire induite par la thrombine ou des affections associées à des cytokines pro-inflammatoires.

6. Composé destiné à être utilisé suivant la revendication 5, ladite maladie étant une maladie inflammatoire choisie entre la pancréatite aiguë, la pancréatite chronique, l'asthme, des allergies et le syndrome de détresse respiratoire de l'adulte.

7. Composé destiné à être utilisé suivant la revendication 5, ladite maladie étant une maladie auto-immune choisie entre la glomérulonéphrite, la polyarthrite rhumatoïde, le lupus érythémateux disséminé, la sclérodermie, la thyroïdite chronique, la maladie de Graves, la gastrite auto-immune, le diabète, l'anémie hémolytique auto-immune, la neutropénie auto-immune, la thrombocytopénie, la dermatite atopique, l'hépatite active chronique, la myasthénie grave, la sclérose en plaques, une maladie intestinale inflammatoire, la colite ulcérative, la maladie de Crohn, le psoriasis et la réaction du greffon contre l'hôte.

8. Composé destiné à être utilisé suivant la revendication 5, ladite maladie étant un trouble osseux destructeur choisi entre l'arthrose, l'ostéoporose et un trouble osseux dû au myélome multiple.

9. Composé destiné à être utilisé suivant la revendication 5, ladite maladie étant une maladie proliférative choisie entre la leucémie myélogène aiguë, la leucémie myélogène chronique, le mélanome métastasique, le sarcome de Kaposi et le myélome multiple.

10. Composé destiné à être utilisé suivant la revendication 5, ladite maladie étant une maladie neurodégénérative choisie entre la maladie d'Alzheimer, la maladie de Parkinson, la sclérose latérale amyotrophique, la maladie de Huntington, l'ischémie cérébrale et une maladie neurodégénérative provoquée par une lésion traumatique, la neurotoxicité du glutamate ou une hypoxie.

11. Composé destiné à être utilisé suivant la revendication 5, ladite maladie étant choisie entre une ischémie/reperfusion dans un ictus ou une ischémie myocardique, l'ischémie rénale, des attaques cardiaques, une hypoxie d'organe et l'agrégation plaquettaire induite par la trombine.

12. Composé destiné à être utilisé suivant la revendication 5, ladite maladie étant une affection associée à l'activation des lymphocytes T ou à des réponses immunitaires pathologiques.

13. Composé destiné à être utilisé suivant la revendication 5, ladite maladie étant un trouble angiogénique choisi entre des tumeurs solides, la néovascularisation oculaire et un hémangiome infantile.

14. Composition comprenant un composé suivant l'une quelconque des revendications 1 à 4 ou un de ses sels pharmaceutiquement acceptables, et un support, véhicule ou diluant pharmaceutiquement acceptable.
